(19) **Europäisches Patentamt**

**European Patent Office**

**Office européen des brevets**

(11) **EP 1 705 468 A2**

(12) **EUROPEAN PATENT APPLICATION**

(43) Date of publication:
 **27.09.2006 Bulletin 2006/39**

(51) Int Cl.:
 ***G01H 1/00*** (2006.01)

(21) Application number: **06004256.1**

(22) Date of filing: **02.03.2006**

(84) Designated Contracting States:
 **AT BE BG CH CY CZ DE DK EE ES FI FR GB GR HU IE IS IT LI LT LU LV MC NL PL PT RO SE SI SK TR**
 Designated Extension States:
 **AL BA HR MK YU**

(30) Priority: **15.03.2005 JP 2005074120**

(71) Applicant: **OMRON CORPORATION**
 **Kyoto-shi, Kyoto 600-8530 (JP)**

(72) Inventor: **Ikuma, Fukui**
 **Omron Corporation**
 **Shiokoji-dori**
 **Kyoto-shi**
 **Kyoto600-8530 (JP)**

(74) Representative: **Kilian, Helmut**
 **Wilhelms, Kilian & Partner**
 **Patentanwälte**
 **Eduard-Schmid-Strasse 2**
 **81541 München (DE)**

(54) **Abnormality detection device and method having a judgment model**

(57) An endurance test is carried out after judgment models are created from normal data obtained from test apparatus while operating stably and presence and absence of abnormality in a target object is judged from waveform data obtained during the test and by using the created judgment models. An aid device creates such judgment models automatically by creating divided waveform data by dividing obtained waveform data into portions of unit time, obtaining characteristic quantities in units of the divided waveform data based on a plurality of the divided waveform data for the unit time. The judgment models are created for the unit time based on the obtained characteristic quantities.

Fig.1

**Description**

Background of the Invention

This invention relates to an inspection apparatus, an aid device for creating a judgment model for an inspection apparatus, an abnormality detecting device for an endurance test apparatus and a method of endurance test. More in particular, this invention relates to the technology of extracting a characteristic quantity from an inputted waveform signal and judging a condition based on the extracted characteristic quantity.

There are technologies for product inspection and apparatus diagnosis in which it is determined whether a target object of inspection is in a normal condition or in an abnormal condition, based on sounds or the like generated by this target object. The apparatus diagnosis is the technology of determining whether a fabrication machine or a production equipment is operating normally or it is about time to require a maintenance work or an adjustment, based on vibrations or sound generated by the fabrication machine or the production equipment itself. Examples of target object to be diagnosed may include NC fabrication machines, semiconductor plants and food plants. The product inspection is to inspect whether a target product is a normal product or a defective product, based on vibrations or sound generated by the product. They are common in that an inspection is carried out based on vibrations or sound.

Let us consider the production inspection as an example. Among the products produced by a production equipment or a production system, there are those incorporating a sound source or a vibration source inside. There are also products that generate a sound or vibrations when they are operated. Household electrical products such as refrigerators, air conditioners and washers contain a component such as a motor and generate vibrations, when activated, due to the rotation of the motor. In the case of an automobile, there are many sound sources and vibration sources such as the engine, the power steering equipment and the transmission.

Some of these sounds and vibrations occur necessarily as a part of the normal operation of the product while some of them occur only when there is an abnormality. Abnormal sounds and vibrations associated with an abnormal condition may be caused by an abnormal contact inside the motor, an abnormality in a bearing for a rotating mechanism, an abnormal contact inside a rotary mechanism, an unbalanced condition of a rotary mechanism or the existence of a foreign object. Explained more in detail, there are abnormal sounds of the kind that would occur only instantaneously such as when a fixed part and a mobile part of a motor contact each other instantaneously. In the case of a rotary mechanism, there are also abnormal noises of the kind that would occur periodically, say, once per rotation of a rotary gear. Examples of noise of this kind include those caused by a chipped gear, a foreign object in the gear box and a spot damage in the bearing.

Examples of noise which gives an unpleasant sensation to a person include a screech which sounds instantaneously only once, mixed in an uniform noise of operation. If the uniform noise is to be expected from the operation of a normal product, the product that causes a screech may be regarded as a defective product.

In the case of porcelain products or products that combine resin parts, they may not include any component that can function as a source of sound or vibrations, but there are situations where the existence of a crack may be examined on such a product. This can be effected by striking the product by a tool such as a hammer to cause a sound. A product without a crack produces a high sound but the produced sound is dull if there is a crack. An examination is possible because of this difference in the produced sounds.

Throughout herein, "sounds" shall include vibrations and "vibrations" shall include sound. In other words, for the sake of convenience, expressions "sound" and "vibrations" will be sometimes used interchangeably.

Sounds associated with an abnormality are not only unpleasant to the listener but also likely to themselves cause a further abnormality. Thus, products that generate such sounds should be separated from normal products by an inspection in the production process. It has therefore been practiced to carry out a so-called sensory test, depending on the five senses of an inspector at a production site, on each product produced by a production equipment or a production system. More in detail, tests by listening and touching to feel the vibrations have been carried out. The sensory tests are defined in Z8144 of JIS (Japanese Industrial Standard).

Sensory tests, depending on the five senses of an inspector, are disadvantageous not only because they require skilled inspectors but also because the test results include large individual variations. Moreover, it is difficult to translate test results into numerical data and hence to manage them. In response to this problem, there have been developed noise inspection devices for carrying out inspection according to a clear quantitative standard. These noise inspection devices are for automating the steps of a sensory test, adapted to measure the vibrations or sounds of a product driving part by means of a sensor and to analyze and inspect the analog signal taken in by the sensor.

Japanese Patent Publication Tokkai 3484665 has disclosed such a device adapted to automatically judge normal and abnormal products from a vibration waveform obtained from a target object. This device is characterized as providing a summary judgment by using a characteristic quantity obtained from a waveform along a time axis and a characteristic quantity obtained from a frequency waveform.

Japanese Patent Publication Tokkai 3103193 describes, on the other hand, another technology according to which a normal range for normal products is initially formed only from normal data obtained from normal products and a product is judged normal if the inspection value obtained therefrom is found to be within this range. A plurality of input data are used to set the normal range by means of a multi-dimensional vector.

When products such as engines, transmissions and tires are being developed, an endurance test is frequently carried out during a trial stage for ascertaining whether or not there are weak components (which break up during the test). During such an endurance test, sensors such as a rotary meter, a temperature sensor and a vibration meter are required to record real-time changes in conditions of a test workpiece (such as a test engine, a test transmission and a test tire). If such a test is not immediately stopped when a portion of the target object being tested breaks down first, this initial damage (primary damage) may cause a secondary damage soon afterwards somewhere else, causing a tertiary damage still somewhere else and other subsequent damages. If the test is stopped only after such series of damages has already occurred and an attempt is made to analyze the test workpiece, it is extremely difficult to identify the primary damage representing the weakest portion. Even if such an identification were possible, it would take a large amount of time and labor.

The weakest component which breaks down first does not always break down suddenly. It is quite often the case that some event occurs as a precursor of the primary damage. For example, before there is a breakdown, there is usually a small abnormality that occurs due to wears or deformations. As the endurance test is continued, such abnormality grows until the component cannot withstand the abnormality and breaks down, resulting in the primary damage. In such a case, the motion of the constituent parts of the test workpiece changes and hence its vibrations change. The driving load, temperature and distortions of the test workpiece will come to change and these changes cause a change in the noise produced by the test workpiece. In other words, these compounded data result in a transmitted sound.

The tone of the sound from an object undergoing an endurance test shows a difference before and after the primary damage. If an inspector experienced in the workpiece stays by the test workpiece during the endurance test and keeps listening to the sound, it may be possible to detect the occurrence of a primary damage by detecting the change in the tone of the sound. In other words, since the inspector does not make judgments by concentrating on a specified kind of data (such as only a specified frequency, only a specified vibration frequency, or the temperature of only a specified control) but from the pattern of an entire frequency range such as the tone of a sound, and this is how the difference between a normal condition and an abnormal condition can be noted. Thus, if judgments are made only on the basis of data of a specified kind, as by inspection systems used in prior art endurance tests, abnormality cannot be detected at the point in time of the primary damage or at the precursor stage foreseeing a primary damage.

Thus, although it is a fact that an abnormality can be detected at the stage of a primary damage or at the stage of a precursor to a primary damage by depending on the inspector's sense of hearing, this is not a practical solution to the problem because many endurance tests last for 24 hours to a week and sensitivity of a person is not constant. An inspector may detect an abnormality at the stage of a primary damage on one occasion but may fail to notice it until the stage reaches a secondary or tertiary damage on some other occasion.

In the case of an inspection apparatus as described in aforementioned Japanese patent publications, adapted to judge normal and abnormal conditions based on a waveform signal of a sound or vibrations, a data waveform for a specified time length from 5 seconds to 30 seconds is taken while a test workpiece is driven and a judgment is made on this obtained waveform. In other words, the judgment is based on the whole of a data waveform of a specified number of seconds obtained by driving the workpiece for a specified length of time.

In the case of an endurance test, by contrast, waveform data lasting for more than 24 hours are continuously inputted and real-time judgments are made on such long data. Thus, conventional inspection apparatus of the type described above cannot be directly used for an endurance test.

For the application in an endurance test, furthermore, since the allowed time length between the occurrence of a damage and stopping the test is very short and an accurate judgment is required from waveform data of a very short period from 0.1 second to 1 second is required, prior art algorithms for determination of normal and abnormal conditions based on waveform data of several seconds cannot easily be used directly.

Summary of the Invention

It is therefore an object of this invention to provide an inspection apparatus, an aid device for creating a judgment model for an inspection apparatus, an abnormality detection device for an endurance test apparatus and a method of endurance test, capable of making normal and abnormal judgments even from waveform data of a short time duration and also capable of detecting an abnormality within a short time even while waveform data are being obtained from a test workpiece that is being driven continuously over a relatively long period of time.

An aid device according to this invention is for creating judgment models for an inspection apparatus for extracting characteristic quantities from an inputted waveform signal and may be characterized as comprising divided waveform data creating means for creating divided waveform data by dividing obtained waveform data into portions of unit time ofN seconds, calculating means for obtaining characteristic quantities in units of the divided waveform data based on a plurality of the divided waveform data for the unit time, and model creating means for creating judgment model for judging condition of said target object for the unit time based on the obtained characteristic quantities. With an aid device thus structured, accurate judgment models can be created even if the unit time is short because different divided waveform data are used.

The divided waveform data creating means may have the function of dividing waveform data into portions of unit time regularly from a specified position. It may also be provided with the function of determining this specified position at random. In either case, many divided waveform data can be collected and hence accurate models can be created especially since one cannot predict in real situations when and where an abnormality should occur.

An aid device according to another embodiment of the invention may be characterized as comprising divided waveform data creating means for creating divided waveform data by dividing obtained waveform data sequentially and regularly into portions of unit time, calculating means for obtaining characteristic quantities in units of the divided waveform data based on a plurality of the divided waveform data for the unit time, and model creating means for creating judgment model, based on the obtained characteristic quantities, for each of areas into which said pattern has been divided by said divided waveform data creating means. Such an aid device is convenient when the action profile of the target object of inspection is periodically repeated.

In the above, the calculating means may be provided with the functions of carrying out a frame division process by further dividing the divided waveform data into frames of a specified size, extracting the characteristics quantities based on the divided frames, and determining this specified size at random. In this way, many frames can be collected and hence accurate judgment models can be created.

An inspection apparatus of this invention is characterized as comprising judging means for carrying out a status judgment on obtained target object of inspection based on a judgment model created by an aid device of this invention.

An endurance test apparatus of this invention is an abnormality detector used for carrying out an endurance test to test endurance of a target object and may be characterized as have judgment model creating function for creating judgment models by collecting sensing data waveform data while the apparatus is running stably immediately after the test is started and assuming such data as being normal data and comprising judgment means for judging whether the target object is normal or abnormal by using the created judgment models and based on waveform data obtained during the endurance test. In the above, the judgment model creating function may be realized by an aid device of this invention.

An endurance test method of this invention may be characterized as comprising the steps of causing a rotary motion for a specified length of time and providing waveform data obtained meanwhile to an aid device of this invention, causing the aid device to create judgment models by using the provided waveform data as normal data, thereafter carrying out an endurance test to judge presence and absence of abnormality based on waveform data that are obtained during the endurance test by using the created judgment models, and outputting an alarm signal if abnormality is detected.

Brief Description of the Drawings

Fig. 1 is a schematic block diagram of an endurance test system according to this invention.
Fig. 2 shows the entire flow of an endurance test carried out according to this invention.
Figs. 3A and 3B, together referred to as Fig. 3, are conceptual diagrams for explaining creation of judgment algorithm and judgment process based thereon.
Fig. 4 is a functional block diagram of the inspection apparatus.
Figs. 5 and 6 are drawings for showing the function of the waveform dividing means.
Fig. 7 shows the inner structure of the dummy NG creating means.
Fig. 8 shows an example of data structure in the characteristic quantity-history database.
Figs. 9, 10 and 11 are drawing for explaining numerization processes.
Fig. 12 is a graph showing an example of frequency pattern of a sound from a test workpiece while it is being driven.
Fig. 13 shows the internal structure of an example of waveform data numerizing means.
Fig. 14 is a flowchart of a portion of the functions of the waveform data numerizing means.
Figs. 15, 16, 17 and 18 show different internal structures of the judgment model creating means.
Figs. 19 and 20 show different internal structures of the threshold setting means.
Fig. 21 is a block diagram of an inspection apparatus of this invention during operation.
Figs. 22A, 22B, 22C, 22D and 22E, together referred to as Fig. 22, show functions of the inspection apparatus of this invention.
Figs. 23A, 23B and 23C, together referred to as Fig. 23, show functions of the inspection apparatus of this invention in another example.
Figs. 24 and 25 are drawings for explaining the function for creating judgment models based of different operation profiles.
Figs. 26 and 27are drawing for explaining another embodiment based on different operating profiles.
Figs. 28 and 29 show examples of display of changes in normalcy

Detailed Description of the Invention

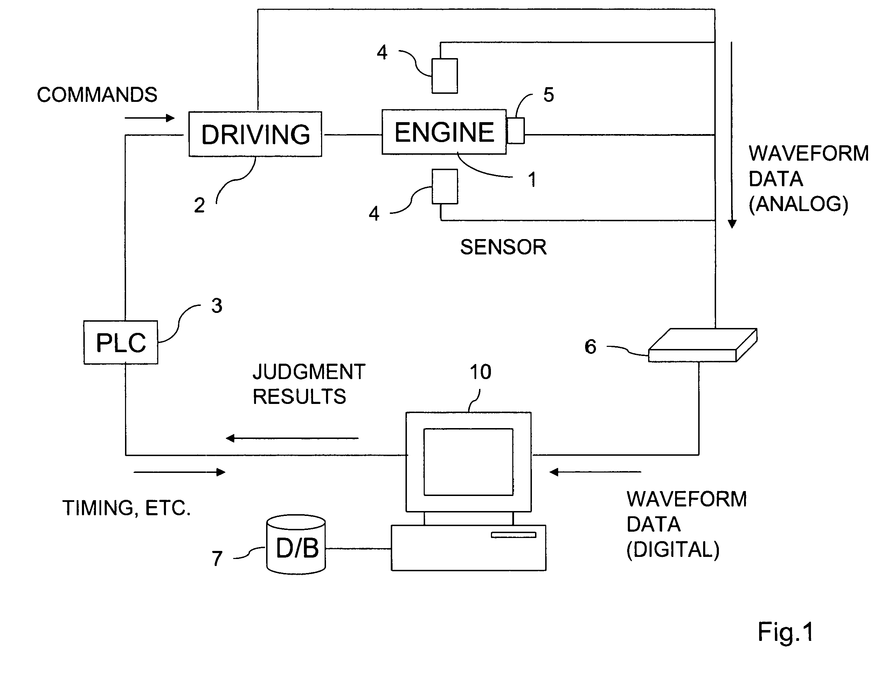

Fig. 1 shows the schematic structure of an endurance test system according to a preferred embodiment of this invention. A driving device 2 such as a motoring bench is connected to the rotary shaft of an engine 1 serving as the object to be tested such that the engine 1 can be forcibly rotated. The driving device 2 is provided with a servomotor and a servo-driver for controlling the rotation of this servomotor, and the servomotor is adapted to rotate at a specified rotary speed according to a control command received from a PLC 3. Since this is a servomotor, it can be rotated at a constant rate and its rotational speed can be increased or decreased accurately. Thus, the engine 1 can be caused to rotate continuously for a relatively long period of time (from one day to two weeks or even longer) at a desired rate.

Microphones 4, as sensors for detecting sound from the engine 1, are disposed around the engine 1, and a vibration sensor 5 for detecting the vibrations of the engine 1 is placed so as to contact a specified part of the engine 1. According to the example shown in Fig. 1, two microphones are used, one above and the other below the engine 1, but the number of microphones to be used and the places where they are set are arbitrary, and the same also applies to the vibration sensor. Neither does the invention require different kinds of sensors such as microphones and vibration sensor to be set for the engine. What is essential is to obtain waveform data from which an abnormality can be detected.

Analog waveform data outputted from the microphones 4 and the vibration sensor 5 are transmitted to an inspection apparatus 10 after being converted to digital data by means of an AD converter 6. The inspection apparatus 10 is further adapted to receive the operation timings from the PLC 3, and waveform data from the monitoring sensors (such as encoders and torque meters) set on the driving device 2 are received from the driving device 2 through the AD converter 6.

The inspection apparatus 10 is provided with a database 7 which stores data such as judgment models and judgment rules for detecting abnormalities and serves to judge the presence and absence of abnormalities in waveforms data received from the AD converter 6 according to judgment models, etc. received from this database 7, transmitting a report on occurrence of abnormality to the PLC 3 if the presence of an abnormality has been detected. If such a report on an abnormality is received, the PLC 3 outputs an instant stop command to the driving device 2 so as to instantaneously stop the rotation of the servomotor of the driving device 2 which is capable of stopping relatively quickly, thereby also stopping the rotation of the engine 1 being tested.

As will be described below, the detection of an abnormality can be made based even on waveform data of only about one second, that is, nearly simultaneously with its occurrence. In the above, examples of the abnormality that is to be detected include not only the occurrence of a primary damage to a component of the engine 1 but also conditions that would lead to a primary damage if the driving were continued in the same manner. Since a judgment of abnormality can thus be made quickly and accurately based on waveform data of only one second, the engine 1 can be stopped also quickly (a little over one second) from the moment of the detection of the abnormality because the detection is communicated to the PLC 3.

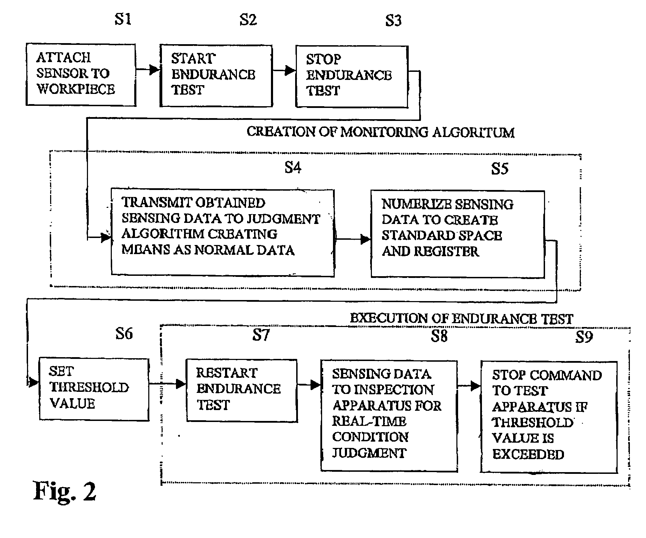

Fig. 2 is a drawing that shows the entire flow of an endurance test carried out according to this invention. The test is started firstly by attaching a sensor to the workpiece (Step S1). According to the example of Fig. 1, the engine 1 is the workpiece to be tested and the sensors 4 and 5 are set at specified positions of the engine 1 after it is set on a driving device 2 such as a motoring bender. The method of attachment depends on the kind of the sensor. Some sensors may be attached directly on the engine 1. Some may be set at a specified distance away. The method of setting these sensors is the same as used in conventional methods of inspecting noise.

After the sensors are set, the endurance test is started (Step S2). This is done by repeating various profiles of motion such as rotating at a certain rate for a specified period of time, accelerating, decelerating or repeating a specified pattern including acceleration, deceleration and motion of a constant speed. While these various profiles are carried out, the sensing data (waveform data) obtained from the various set sensors (microphones, vibration sensor and/or monitoring sensor mounted to the driving device) are converted by the AD converter 6 into digital data and then inputted to the inspection apparatus 10. The waveform data thus received by the inspection apparatus 10 may be stored in the database 7 or in a primary memory means within the inspection apparatus 10. Basically, since the engine 1 that is tested is a normal product, the waveform data which are obtained immediately after the beginning of the endurance test may be regarded as normal waveform data expected from a normal product.

After a certain time has passed from the beginning of the endurance test, it is stopped (Step S3) and the sensing data that have been obtained thus far are transmitted as normal data to a judgment algorithm creating means (or judgment model creating aid device) mounted within the inspection apparatus 10 (Step S4). The judgment algorithm creating means, upon receiving the normal waveform data, converts them into numerical data (or "numerizes") to create a standard space and a judgment model that define a normal area and registers in a judgment algorithm (Step S5). This judgment algorithm is one of the application programs installed in the inspection apparatus 10 and is for realizing the abnormality detection means (function) for carrying out condition judgment (or the judgment of whether an abnormality has occurred or not) at the time of endurance test. In reality, this "registration" may be effected to the database 7.

In the case of a workpiece (such as an engine) that needs a warm-up operation, sensing data are obtained several times as initial normal condition after the end of a warm-up operation, and the judgment algorithm (judgment model) is created based on these.

Strictly speaking, waveform data of a normal product vary not only from one engine to another but also depending on the combination of the driving device 2 and the engine 1, the manner of connection between them and the environmental conditions. Thus, every time an endurance test is carried out on an engine according to this invention, it is operated for a specified period of time before a continuous operation for a long time is carried out and normal waveform data are obtained with the environmental condition taken into consideration for creating a standard space and a judgment model.

After Steps S4 an S5 are completed and a monitoring algorithm has been created, a threshold value for the judgment of abnormality from the judgment model (or a value for defining and detecting an area separated from the standard space) is set (Step S6). This threshold value is registered in the judgment algorithm (or the function for carrying out this algorithm) in the inspection apparatus 10.

Now, the actual endurance test is started, or the endurance test that was stopped in Step S2 is restarted (Step S7) by operating the engine continuously for a long time according to a required profile. During this time, various sensing data (waveform data) that are obtained from the various sensors are provided to the judgment algorithm in the inspection apparatus 10 to carry out real-time condition judgment (Step S8). If the threshold value is exceeded, an instant stop command is outputted to the tester (the driving device 2) (Step S9).

In summary, the inspection apparatus 10 is capable of judging the presence and absence of an abnormality based of waveform data corresponding to each time unit and reports to the PLC 3 on the detection of an abnormality as a judgment result if the threshold value is exceeded, say, by changing the judgment result bit from "0" to "1". Upon receiving this report, the PLC 3 outputs an instant stop command to the driving device 2. As a result, the servomotor in the driving device 2 is stopped instantaneously and the corresponding endurance test, too, can be immediately stopped before a secondary damage can occur. Thus, if the primary damage has already occurred, the component that has been damaged can be easily identified. If the operation is stopped before the occurrence of the primary damage, the weakened component likely to become damaged soon can be identified relatively easily. Thus, the time required for the endurance test and for its analysis can be significantly reduced.

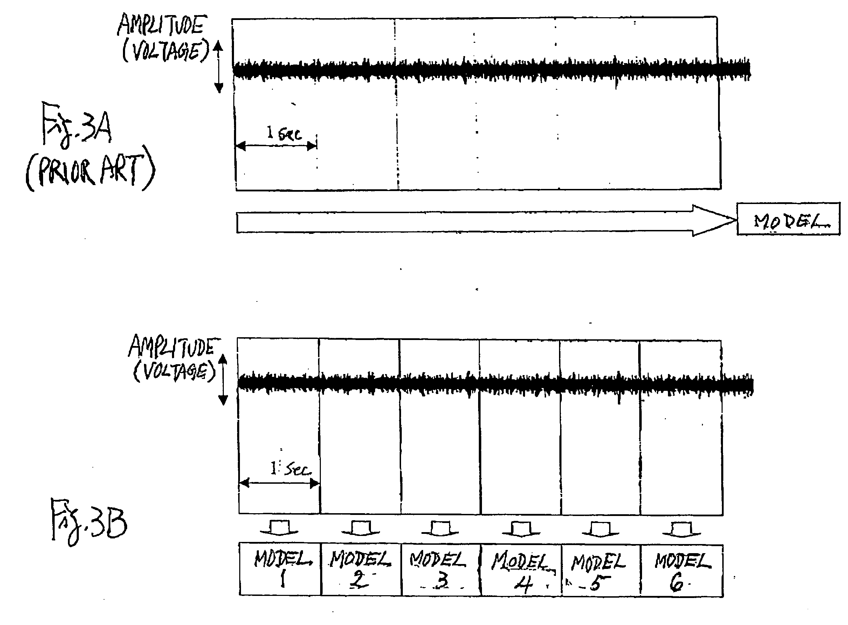

Fig. 3A is a diagram for showing the concept of judgment model in the case of a prior art in-line noise inspection apparatus. In the case of an in-line inspection apparatus, a judgment model is created based on normal sound tone over a certain period of time (such as 6 seconds according to the example) and a judgment is made based on this judgment model. If this prior art judgment algorithm for an in-line inspection apparatus is directly used, since the judgment model is created from waveform data for 6 seconds, the occurrence of an abnormality at some time during this period of 6 seconds can be ascertained but the inspection apparatus cannot be stopped at the same timing as its occurrence. This is because prior art in-line inspection apparatus are expected only to judge whether the target object of inspection is normal or abnormal and not required to judge at what portion of the series of waveform data the abnormality has occurred (or to stop the inspection apparatus instantly).

According to this invention, after waveform data for a normal condition have been obtained, it is not that the judgment model is created from the whole of the obtained waveform data but the waveform data are divided, as shown in Fig. 3B, into time segments (each of 1 second in this example). A judgment model is created for each time segment using the sound tone of a normal condition at that time and a judgment algorithm based on these judgment models is carried out. Thus, presence or absence of an abnormality can be judges for each period of 1 second and there is no need to wait for 6 seconds to know the judgment result, although some length of time will be spent for outputting a stop command.

Although Fig. 3B shows an example wherein waveform data for 6 seconds are divided into portions each corresponding to one second to create Models 1-6 individually for the six time segments, if the endurance test is for the purpose of investigating the status change over a long period time by a rotary motion at a constant speed and the external conditions do not significantly change in the meantime, for example, a judgment model for a one-second period may be created from Models 1-6 obtained as above. In the case of an endurance test wherein accelerations and decelerations are repeated according to a specified pattern (which may also include a constant-speed motion), models may be created according to such different external conditions inclusive of the speed such that judgments can be made from these models. What is of importance is that the judgment model is not created from the whole of waveform data that have been obtained but individual models are created by dividing the time of the waveform data into segments. The unit time need not be fixed but may be varied, say, to 0.5 seconds or over 1 second. This time length may be determined according to the required (allowed) time length from the occurrence of an abnormality (or the detection time) until when the device should be stopped.

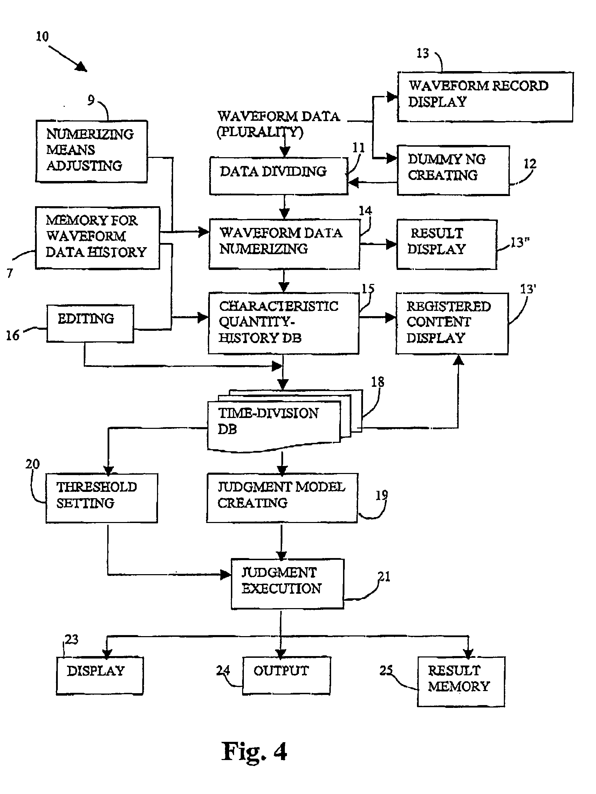

The internal structure of the inspection apparatus 10 is described next with reference to Fig. 4 which is its functional block diagram for creating a judgment algorithm. The inspection apparatus 10 is adapted to receive waveform data from various measurement devices such as microphones and vibration sensors provided to an engine or a driving device therefor. Waveform data of a plurality of kinds may be obtained from a plurality of positions and hence the inspection apparatus 10 may be adapted to handle waveform data of a plurality of kinds.

The waveform data obtained by Steps S2 and S3 of Fig. 2 are provided to data dividing means 11, dummy NG creating means 12 and waveform record display means 13. The waveform dividing means 11 is for dividing received waveform data into a plurality of parts, and a characteristic quantity is obtained for each divided waveform data portion and a final judgment model is created.

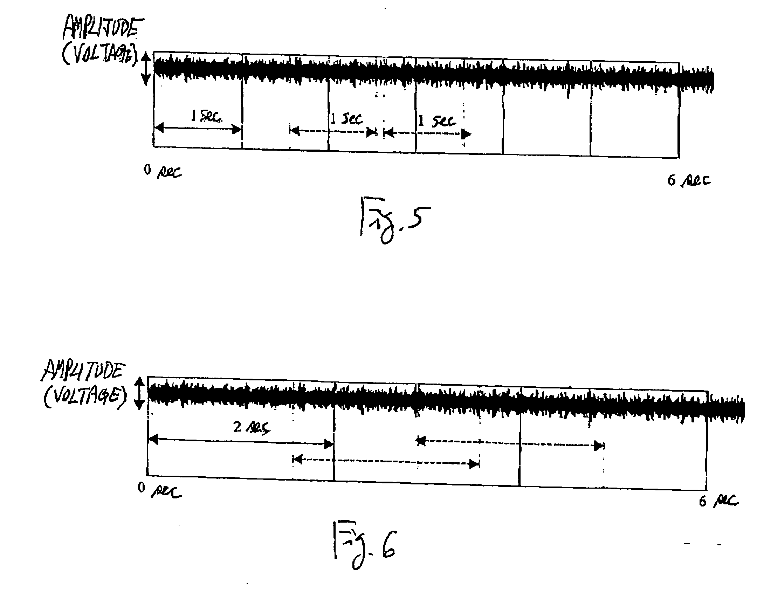

Fig. 5 shows an example of how the waveform dividing means 11 may divide obtained continuous waveform data into units of N seconds (N = 1 in the illustrated example) which may be determined as the time period required by the user as the time necessary from the moment when an abnormality occurs until when the target device must be stopped. Fig. 5 shows a portion corresponding to 6 seconds of waveform data obtained from a continuous test of rotation at constant speed at constant torque.

If a judgment mode created from divided waveform data as explained above is used, an abnormality can be detected within one second of its occurrence and the time required for instantly stopping becomes about one second.

One of the methods for dividing into time segments is to sequentially and regularly dividing from the beginning in units of N=1 second, as shown by solid lines in Fig. 5. By this method, six portions of divided waveform data are extracted and a characteristic quantity is extracted, as will be explained below, for each of these portions. Since all of the waveform data that exists during these six seconds exist in at least one of the divided waveform data portions, an effect of creating a characteristic quantity is experienced somewhere in these data.

Another method for dividing into time segments is to choose the starting time at random to extract a specified number of waveform data portions such that extracted waveform data portions may overlap with each other or may leave an area not belonging to any of the divided waveform data portions. Even if such areas not belonging to any may exist, there is no problem because it will belong to some of the divided waveform data by the first process. For example, segments from 0.7 seconds to 1.7 seconds, from 0.75 second to 1.75 seconds, from 0.9 seconds to 1.9 seconds and from 0.5 seconds to 1.5 seconds may be extracted but each extracted waveform data portion is for one second.

Fig. 6 shows another example where N = 2 seconds. In this case, too, divisions may be made regularly and sequentially from the beginning as shown by solid lines or by starting at random by broken two-headed arrows to extract a specified number (three in this example) of waveform data portions. The maximum number ofN is of course the length of the obtained waveform data (6 seconds in this example).

Even if the time required by the user between the occurrence of an abnormality and the time of instant stopping is 1 second, there may be kinds of abnormality that may not be detectable by divided waveform data corresponding to one second but require a characteristic quantity based on divided waveform data corresponding to a longer time period such as 2 seconds. In such a situation, it may take about 2 seconds to stop the motion after the detection of an abnormality which is longer than the required time length by the user. Even if such a situation may be considered, it is far better than waiting for 6 seconds according to prior art technologies and the identification of the source of abnormality can be detected more easily. In actual applications, the value ofN may be set at random. N may be an integer but need not be an integer.

If it is desired to stop one second after the detection of an abnormality, a judgment model created from waveform data cut out every one second should basically be used as the main model. If data with a trend for a span over one second are to be used for the judgment, however, it is preferable to use a judgment model created from waveform data corresponding to such a length of time. It also goes without saying that N may be freely selected and a plurality of databases and judgment models may be prepared.

Thus, judgment rules (judgment models) created by setting N to be longer than 1 second do not contribute to the instantaneous stopping within one second but since they have the merit of being able to incorporate data with a long-time trend for judgment, it is preferable to incorporate judgment models created from waveform data based on various time lengths.

The dummy NG creating means 12 is for deforming normal waveform data obtained by executing Steps S2 and S3. Dummy NG data created by this dummy NG creating means 12 are provided to the waveform dividing means 11. In the case of the example described above, sample data related to a normal product can be obtained but no sample data related to abnormal ("no good") products can be obtained. This is because the data that are initially obtained in an endurance test are presumed to be normal data and the purpose of the test is to detect the occurrence of abnormality caused by a continuous operation over a long period of time.

Thus, NG data are virtually created by obtaining normal sample data (waveform data) and based on these obtained normal waveform data. Dummy NG data thus created may be used for evaluating a created judgment algorithm. For example, the dummy NG creating means 12 may provide dummy NG data to each of the means on the downstream side and after making judgments based on them, the accuracy of a created recognition algorithm can be estimated, depending on whether a judgment as an abnormality can be correctly made or not. In other words, it can be used for checking whether a change that has been made from the initial (normal) condition can be correctly detected by the judgment model.

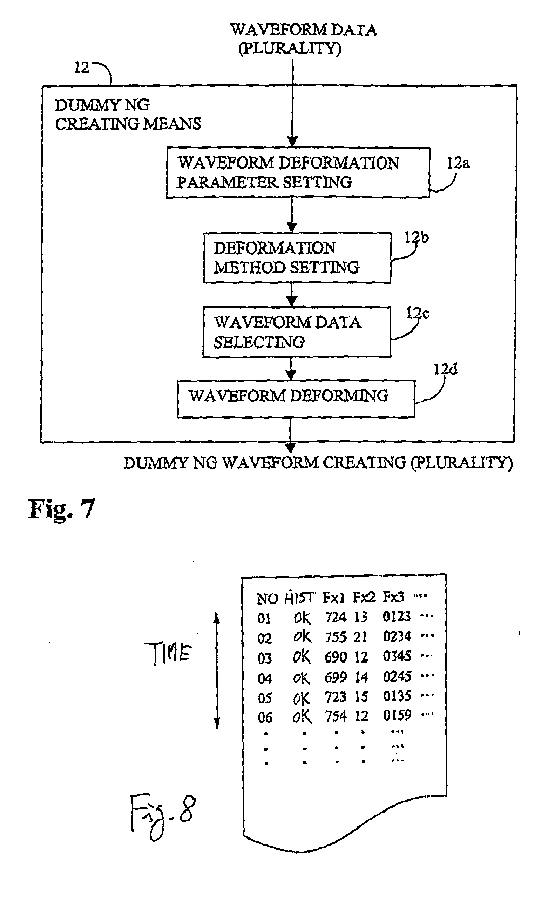

Fig. 7 shows the inner structure of the dummy NG creating means 12, provided with a waveform deformation parameter setting means 12a on the input side for setting parameters for deforming inputted waveform data.

Examples of parameters set by this waveform deformation parameter setting means 12a include those related to (1) syntheses of abnormal mode waveforms of waveform library (syntheses of eccentric abnormal waveforms and shock-wave waveforms), (2) nth order amplification of characteristic frequency of driving conditions (increasing 1st-4th order amplitudes of rotational and engagement frequencies by a factor of 1.5), (3) amplification of specified or random frequency (increasing amplitudes of frequencies 500-1000Hz by a factor of 1.2), (4) FM and AM modulations and (5) phase shifting (shifting the phase of original waveform slightly and synthesizing with the original waveform).

In the above, the waveform synthesis of (1) means synthesizing abnormal waveforms that are not generated from normal waveforms such that waveforms different from normal waveforms appear at overlapped portions of the synthesized waveform from the effects of the synthesized abnormal waveform portions and become abnormal data. The parameters of (2) appear, for example, in the case of a gear as sounds or vibrations that appear at a time of abnormality at a specified frequency, depending on the frequency of engagement. The frequency of engagement can be calculated from the number of cogs and the rotational frequency. Thus, if the nth-order amplitude of each frequency is increased (by increasing the power of a normal product), abnormal data different from normal data are obtained. Detailed explanations will be omitted but waveform data not obtainable normally can be created in other situations.

Parameters that have been set are provided to a deformation method setting means 12b at the next stage together with waveform data. The deformation method setting means 12b is for setting a method of deforming waveform deformation parameters according to a list of experiment planning and its examples include two standards of ON and OFF for waveform synthesis, three frequency standards with respect to a specified frequency and three standards (1.2 times, 1,5 times and 2 times) regarding amplification. Such parameter values for the deformation of waveforms are set.

The dummy NG creating means 12 further includes a waveform data selecting means 12c for randomly selecting as many waveform data as there are experiments in the planning list and a waveform deforming means 12d for changing these selected waveforms according to the quantities of deformation selected by the deformation method setting means 12b based on the planning list. Thus, dummy NG waveforms are finally created and added as waveform data to normal waveform data. Judgment of normal and abnormal can be made also based on these waveform data and if judgments as abnormal product can be made thereby, this means that the reliability of the inspection algorithm of the inspection apparatus 10 has been improved.

The waveform record display means 13 is for displaying waveform data that have been taken in from the various measuring devices or record waveform data which have been stored in recording means. Since the inspection apparatus 10 may comprise a multipurpose personal computer or the like, the waveform record display means 13 may be realized by the display monitor of such a personal computer. In the case of an endurance test, continuous waveform data are taken in and real-time abnormality judgment is carried out. In ordinary work situations, therefore, the waveform record display means 13 display waveform data inputted on real-time continuously through. Since waveform data thus taken in continuously are stored in the database 7 or in some other memory means, if an abnormality occurs and the operations are stopped for carrying out an analysis, such stored waveform data may be read out for a display for the purpose of the analysis. In the above, "waveform record" includes both waveforms to be recorded and waveforms that are recorded.

The divided waveform data that have been divided by the data dividing means 11 are provided next to a waveform data numerizing means 14 which serves to "numerize" given divided waveform data (or to convert into numerical data) and to express them in terms of a characteristic quantity.

The waveform data numerizing means 14 is connected to a numerizing means adjusting means 9 and a history recording means for waveform data (database) 7. The numerizing means adjusting means 9 is for adjusting the parameters of characteristic quantities extracted by the waveform data numerizing means 14. It also has the function of providing commands for such parameter adjustments to the waveform data numerizing means 14 and serves to carry out band area division setting, frame division setting and frame variation number setting.

The waveform data history recording means 7 is for recording waveform data such as the sound that is generated when the engine 1 is forcibly driven to rotate by means of the driving device 2. Normal and abnormal judgment results may also be recorded with correlation. Recorded waveform data may be reproduced and judged and results of judgments may be corrected manually.

The waveform data numerizing means 14 is for extracting a specified characteristic quantity from given divided waveform data. Examples of characteristic quantity to be extracted include root-mean-square (RMS) values indicative of average vibration level and magnitude and many other kinds. Such a characteristic quantity is created for each unit time (N) when conditions such as divided waveform data are created. Although not illustrated, they are created for each of profiles of operation such as environmental temperature, rotation rate and conditions of acceleration and deceleration. The waveform data numerizing means 14 can output the numerized results of waveform data (that is, each obtained characteristic quantity) to a result display means 13".

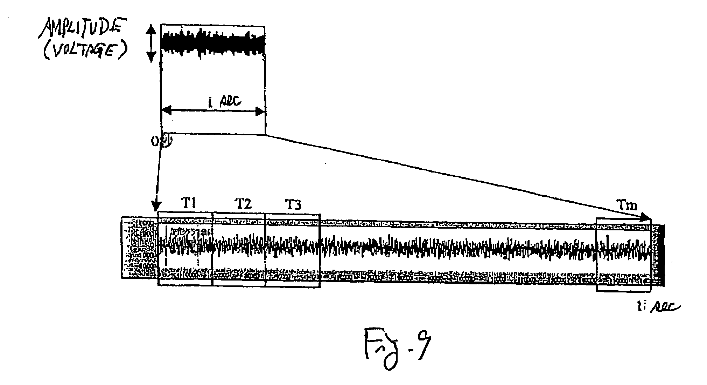

In the case of a motion at a constant speed, characteristic quantities are obtained in units of divided waveform data with the same value of N. If N=1, for example, characteristic quantities are obtained for each of divided waveform data extracted each second by the methods of (1) and (2). Judgment results (normal or abnormal) and the characteristic quantities obtained by the waveform data numerizing means 14 are correlated and stored in a characteristic quantity-history database 15. The characteristic quantities and history are correlated by record numbers as they are stored as shown, for example, in Fig. 8. The column for history (normal or abnormal (with kind of abnormality)) stores data (history type) received from the waveform data history recording means 7 and characteristic quantities received from the waveform data numerizing means 14 are stored in the subsequent columns. When the model (judgment algorithm) is created, the column for history (HIST) is all normal (OK).

In the example shown in Fig. 8, columns Fx1, Fx2, etc. contain characteristic quantities corresponding to the divided waveform data identified by the record numbers. For example, Record No. 1 indicates the characteristic quantity corresponding to the first divided waveform data, Record No. 2 indicates the characteristic quantity corresponding to the next divided waveform data, ..., and Record No. 6 indicates the characteristic quantity corresponding to the last divided waveform data. Although not shown in Fig. 8, the characteristic quantities corresponding to the divided waveform data according to Method (2) with random starting positions are recorded thereafter.

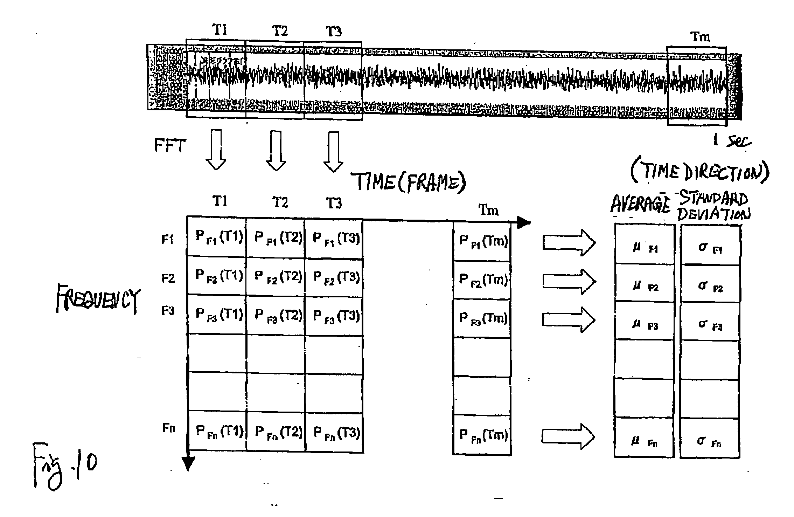

The process for extraction described above is carried out based on the divided waveform data divided along the time axis but characteristic quantities related to the frequency axis such as obtained by FFT or order transformation may be extracted because target waveform data on which FFT is carried out are also divided waveform data. Fig. 9 shows an example of a frame division (into m parts) carried out on a divided waveform data portion with a specified frame size. A frame size less than N seconds (N = 1 in this example) is selected at random. Because of the digital processing, this number is selected to be a power of 2.

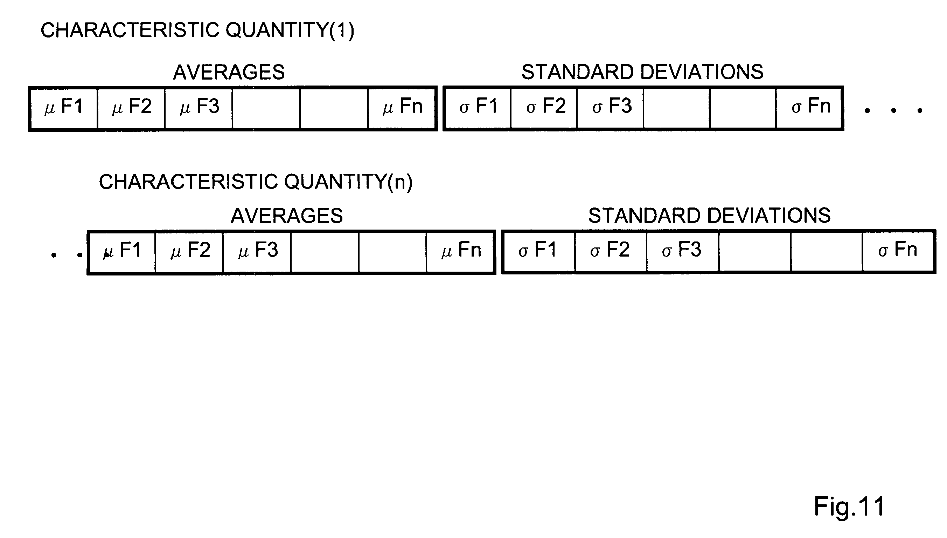

FFT is carried out for each frame to obtain frequency components for all frequency band areas (from F 1 to Fn) such that frequency components are obtained for frequency band areas (from F 1 to Fn) and frames (from T 1 to Tm) in a matrix form as shown in Fig. 10. From this matrix, average power values and standard deviations in the direction of time are calculated for each frequency band area. The averages and the standard deviations thus obtained are arranged in a single row as shown in Fig. 11, and this is referred to as Characteristic Quantity (1).

Next, the frame sizes are redefined at random and the processes as described above are repeated by using these redefined frames to obtain Characteristic Quantity (2). This is repeated for a specified number (n) of times and final characteristic quantity is obtained by arranging all of them in a row horizontally as shown in Fig. 11. Logarithmic conversion may be effected on this row to define the final characteristic quantity.

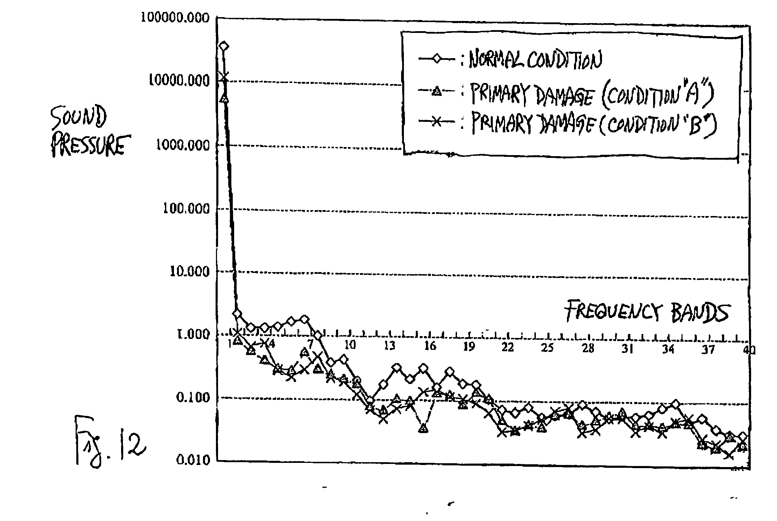

Fig. 12 is a graph showing, as a frequency pattern, the sound (tone) of a test workpiece while it is being driven by dividing a selected frequency band area into 40 band parts. The auditory sense of a human is different from a measurement sensor, sensing not just one specified frequency component but the entire frequency band areas in the audible range as a certain pattern. For this reason, if power of whichever frequency band area is changed, it is recognized as a change in the sound tone. If the purpose is to detect a damage of a specified kind in a product as in the case of a prior art inspection apparatus for an in-line inspection, a filtering process may be carried out for extracting a specified frequency as a preliminary process because a judgment may be possible on the basis of such a specified frequency. In the case of an endurance test, however, there is no way to predict in which of the components a primary damage may take place and hence in which frequency range a change in sound will take place. According to an embodiment of this invention, therefore, a characteristic quantity is obtained by regarding the entire frequency band area as the target of inspection for creating a judgment model such that the entire frequency band area will be recognized as a pattern.

In summary, it is not that only one selected frequency is considered according to this invention but the whole of the frequency band area (from F 1 to Fn) is considered and the frequency pattern is expressed in terms of numbers by some means in order to simulate the human auditory sense. Since sound is a form of vibrations, it goes without saying that the basic principle of this invention is applicable generally to cases with vibrations. For examples, vibrations may be sensed by means of a vibration sensor (or an acceleration sensor) and if this waveform is reproduced by means of a speaker, a change similar to that experienced with a microphone can be sensed by a human. The same is true with a temperature sensor or a distortion gauge instead of an acceleration sensor.

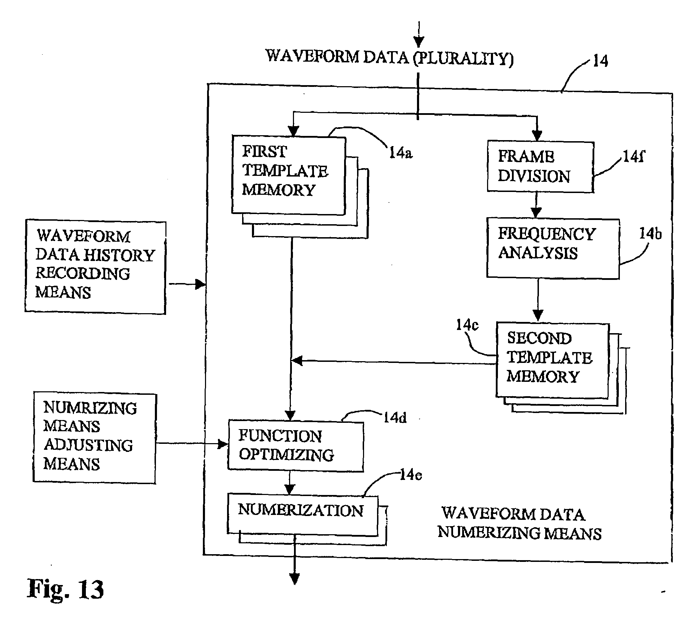

Fig. 13 shows the internal structure of an example of waveform data numerizing means 14. As shown, this waveform data numerizing means 14 is provided with a first template memory 14a for storing divided waveform data obtained from the waveform dividing means 11 and a frame division part 14f that carries out frame division processes on each of the divided waveform data. The functions of the frame division part 14f include, as explained above with reference to Fig. 9, selecting frame sizes at random and dividing each of divided waveform data into frames according to the selected frame sizes. Such frame division processes are repeated for a specified number of times while changing the frame sizes. The waveform data created by the frame division part 14f are received by a frequency analysis part 14b which is adapted to carry out a frequency analysis process of a kind explained above with reference to Fig. 10, such as FFT and order transformation, on the provided waveform data in units of frames. The characteristic quantity data thus obtained are stored in a second template memory 14c. Characteristic data of a plurality of kinds extracted from the same divided waveform data are stored in these template memories 14a and 14c. These characteristic quantity data are each obtained by a specified function.

Items that are obtained by the waveform data numerizing means 14 by using these specified functions are usually predetermined such as the average value and the maximum value although other items are sometimes added but these functions contain adjustable parameters such as coefficients and constants and the accuracy of judgment (of detection of abnormality) improves if these parameters are set appropriately. This means that the accuracy may be adversely affected if the choice is made incorrectly. Conventionally, such parameters were set by experienced workers by a method of trials and errors. The present invention does not exclude selection of parameters by a trial-and-error method but the inspection apparatus 10 automatically optimizes the parameters according to a preferred embodiment of the invention and the optimally set parameters are used to numerize waveform data (to extract characteristic quantities).

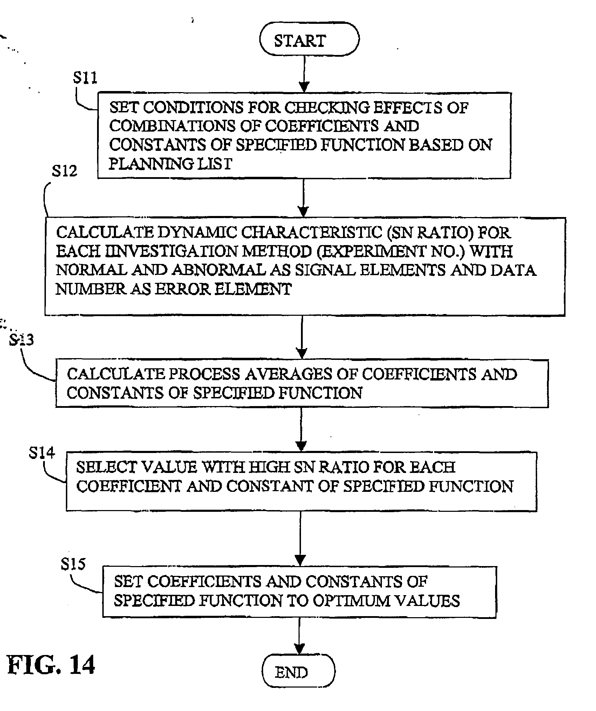

Explained more in detail, the waveform data numerizing means 14 includes a function optimizing part 14d for preparing and optimizing a specified function to be used for numerizing waveform data by changing various parameters according to commands from the numerizing means adjusting means 9. Fig. 14 is a flowchart of functions carried out by the function optimizing part 14d.

The function optimizing part 14d starts by checking the effects of combining coefficients and constants of a specified function and sets investigation conditions based on the list of experiment planning (Step S11). This is done by setting a plurality of combinations of coefficients and constants according to a command from the numerizing means adjusting means 9 to create a planning list in correlation with the investigation conditions (investigation methods). Next, a dynamic characteristic (SN ratio) is calculated for each investigation method (Experiment No.) with normal and abnormal as signal elements and the data number as error element (Step S12). This is done by obtaining characteristic quantities for a plurality of given waveform data by using the specified function set by the parameters (coefficients and constants) prescribed by each investigation method and calculating the distance between the group of characteristic quantities (numerical values) indicating OK (normal products) and the group of characteristic quantities (numerical values) indicating NG (abnormal or defective products).

Next, process averages of coefficients and constants of specified functions are calculated (Step S 13) and the value with high SN ratio is selected for each of the coefficients and constants of the specified functions (Step S14). Parameters (coefficients and constants) of specified functions are determined from these selected values and the specified functions using these coefficients and constants are set as being optimum (Step S 15). Evaluations and setting of parameters of specified functions are carried out for each specified function, that is, for each characteristic quantity.

Optimized specified functions are transmitted to a numerization processing part 14e which serves to use the optimized specified functions that have been set to numerize waveform data and to output the obtained characteristic quantities. These outputted characteristic quantities are stored in the waveform data history recording means 7.

The data stored in the characteristic quantity-history database 15 can be displayed on a registered content display means 13' or changed by operating an editing means 16. This registered content display means 13' and the aforementioned waveform record display means 13 may be physically realized by a single monitor. The data that are stored in the characteristic quantity-history database 15 become stored in a time-division database 18 each of the divided times (N seconds). The data stored in this time-division database 18 are outputted to the registered content display means 13' to be displayed. The editing means 16 is adapted to delete and edit the data stored in the time-division database 18.

Each of the data recorded in this time-division database 18 is transmitted to a judgment model creating means (for each time) 19 on the next stage for each time division. This judgment model creating means 19 creates, based on the characteristic quantity for each time, a judgment formula for judging whether or not target object waveform data (characteristic quantity) being inspected agree with the individual history type, that is, a judgment formula for making a normal judgment based on normal data of normal history type.

The condition judgment formula may be of any form such as the Mahalanobis' distance type, the Euclid distance type, the normal-abnormal comparison type, the neural network type and the fuzzy method using membership function. As will be explained below, it may be created automatically and also manually in a conventional way.

In the case of a test at a constant speed, only one kind of models is created but judgment models are created from data corresponding to several seconds. This is to say that models are created from characteristic quantities of a plurality of waveform data divided into seconds. The judgment models (judgment rules) created here are those for defining a normal range from the average and the standard deviation of each characteristic quantity (say, as within $\pm$ $3\sigma$ of the average) and judging as normal or abnormal according to the degree of distance from this normal range. The judgment model may be adapted to firstly obtain a normal range for each characteristic quantity, to obtain each characteristic quantity from waveform data obtained each second, to determine whether or not each of the characteristic quantities is within the aforementioned normal range (within a specified distance) and to judge as abnormal if there is even one (or a specified predetermined number) that is abnormal among the characteristic quantities. Presence or absence of abnormality may be judged by preliminarily considering a multi-dimensional vector representing each characteristic quantity and based on the distance between this multi-dimensional vector and another multi-dimensional vector obtained from the target waveform data.

Since judgment is initially given according to this invention by a judgment execution means 21 based on judgment formula created only on normal data, only data with normal history are stored in the characteristic quantity-history database 15 and only data with normal history type shown in Fig. 8 are stored in the time-division database 18. Thus, the judgment model creating means 19 creates judgment formulas for making normal judgments to be set in the judgment execution means 21.

Since characteristic quantity values are in the form of a row vector such as:

Normal = f(CV1, CV2, ···, CVn),

the normal level is treated in a normalized form such as "1" (although it may of course be other than "1"). As each characteristic quantity value comes to take up a value different from its initial value immediately after the beginning of an endurance test, the value of f(CV1, CV2, ..., CVn) ceases to be close to 1. Thus, it can be judged from the value of f(CV1, CV2, ..., CVn) whether or not the situation is close to being normal.

Examples of algorithm for calculating the difference from a normal product by using the expression such as f(CV1, CV2, ..., CVn) include methods using the Euclid distance, Mahalanobis distance and the neural network model.

Each of the data recorded in the time-division database 18 is transmitted also to a threshold setting means 20 which is for setting a threshold value for determining whether or not the result obtained by a calculation by using a judgment formula with a characteristic value obtained based on waveform data obtained from the engine 1 matches its history type. The threshold value set thereby is transmitted to the judgment execution means 21.

As a judgment algorithm (judgment model) is created, the judgment execution means 21 uses the judgment formula and the threshold value thus set to make a normal-abnormal judgment, based on waveform data (characteristic quantities) of a given target of inspection. The result of this judgment is not only outputted through a display means 23 or an output means 24 but also stored in a result memory 25. The result memory 25 stores not only condition judgment results but preferably also judgments (history) made by persons, waveform data and characteristic quantities in a correlated form. The display means 23 may be physically the same as the aforementioned waveform record display means 13.

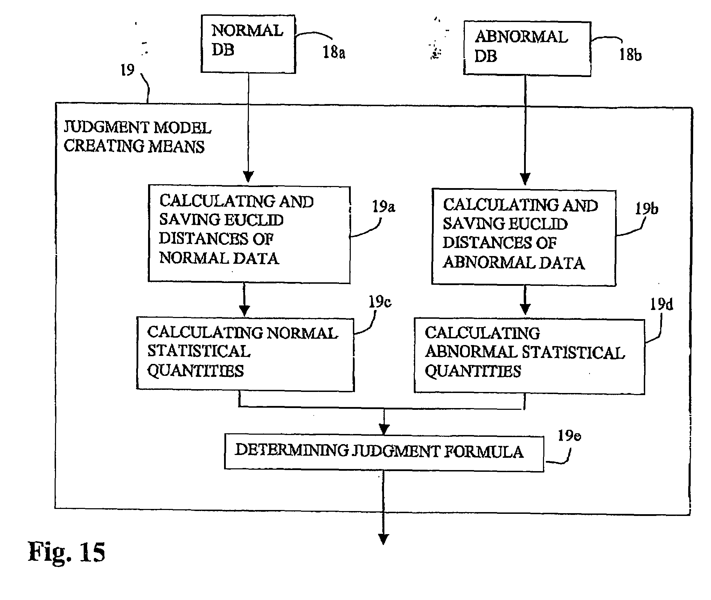

As stated above, the judgment model creating means 19 may be of various kinds. Fig. 15 shows the internal structure that it may assume for carrying out the Euclid distance method. First, characteristic quantity data for each history kind stored in normal database 18a and abnormal database 18b of the time-division database 18 (created by the dummy NG creating means 12) are transmitted to corresponding calculating-saving means for Euclid distances 19a and 19b. The Euclid distance calculating-saving means for normal data 19a obtains Euclid distances based on the obtained normal data (characteristic quantities) by calculating the square root of the sum of the squares of these characteristic quantities and have them saved. The Euclid distance calculating-saving means for abnormal data 19b carries out similar operations except that it is abnormal data that are processed and have the results of the operations saved. If the abnormal data are separated according to the type of abnormality, Euclid distance is calculated for each type of abnormality and saved.

The Euclid distance of each data thus calculated and saved as explained above is transmitted to corresponding statistical quantity calculating means 19c or 19d. The normal statistical quantity calculating means 19c is for calculating normal statistical quantities and calculates statistical quantities of Euclid distances of a plurality of given normal data such as maximum, average and standard variation values. Similarly, the abnormal statistical quantity calculating means 19d is for calculating abnormal statistical quantities and calculates statistical quantities of Euclid distances of a plurality of given abnormal data such as maximum, average and standard variation values. In this case, too, they may be obtained for each of abnormality types.

These statistical quantities obtained by each of the statistical quantity calculating means 19c and 19d are transmitted to a judgment formula determining part 19e which compares the maximum of the normal statistical quantities obtained by the normal statistical quantity calculating means 19c (the maximum normal value) and the minimum of the abnormal statistical quantities obtained by the abnormal statistical quantity calculating means 19d (the minimum abnormal value) to examine whether or not (the maximum normal value) < (the minimum abnormal value). If this inequality is satisfied, the formula for obtaining the Euclid distance of the characteristic distance currently set is judged to be correct and is set in the judgment execution means 21. Thus, the judgment execution means 21 calculates the Euclid distance by obtaining the square root of the total sum of the given characteristic quantities.

If the inequality given above is not satisfied, since it is concluded that the specified functions for calculating the characteristic quantities were not appropriately set, the judgment formula determining part 19e makes a request to the numerizing means adjusting means 9 to modify the parameters (coefficients and constants of the specified function). Upon receiving this request, the numerizing means adjusting means 9 sets different values to the coefficients and constants of the specified function. Since the specified function is thereby changed, the characteristic quantity numerized based on the modified specified function are changed and the statistical values also change. As this process is repeated, conditions satisfactory to the judgment formula determining part 19e come to be created.

If the judgment model creating means 19 is of the kind calculating the Euclid distance as described above, the function for calculating Euclid distance of normal data (the square root of the total of the squares of the characteristic quantities) is specified to the judgment execution means 21.

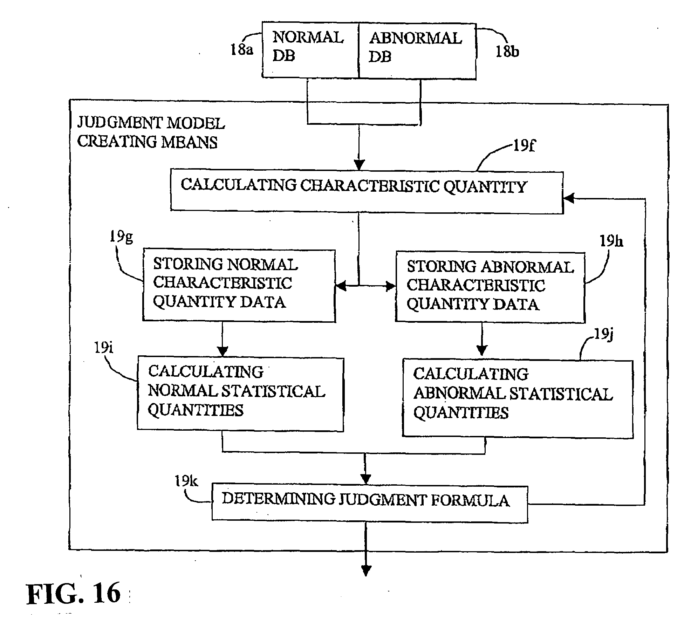

Fig. 16 shows another structure of the judgment model creating means 19 adapted for the normal-abnormal comparison method. By this method, characteristic data stored in the normal database 18a and the abnormal database 18b of the time-division database 18 are transmitted to a characteristic quantity calculating means 19f adapted to select a specified function and to use it to calculate characteristic quantities of the data stored in these databases. Characteristic quantities of normal data are stored in a normal characteristic quantity data storing means 19g and characteristic quantities of abnormal data are stored in an abnormal characteristic quantity data storing means 19h.

These characteristic quantities of data thus calculated and stored as described above are transmitted to the individually corresponding statistical quantity calculating means 19i and 19j. The normal statistical quantity calculating means 19i is for calculating statistical quantities of characteristic quantities of normal data and calculates maximum, average and standard deviation values of the characteristic quantities of the plurality of given normal data. Similarly, the abnormal statistical quantity calculating means 19j is for calculating statistical quantities of characteristic quantities of abnormal data and calculates maximum, average and standard deviation values of the characteristic quantities of the plurality of given abnormal data.

The statistical quantities obtained by the statistical quantity calculating means 19i and 19j are transmitted to a judgment formula determining part 19k, which compares the maximum of the normal statistical quantities obtained by the normal statistical quantity calculating means 19i (the maximum normal value) and the minimum of the abnormal statistical quantities obtained by the abnormal statistical quantity calculating means 19j (the minimum abnormal value) to examine whether or not (the maximum normal value) < (the minimum abnormal value). If this inequality is satisfied, the specified function extracted by the characteristic quantity calculating means 19f is judged to be correct and is defined as such in the judgment execution means 21. Thus, the judgment execution means 21 calculates the Euclid distance by obtaining the square root of the total of the squares of the given characteristic quantities and judges the condition based on whether this Euclid distance is above the threshold value or not.

If the inequality give above is not satisfied, since it is concluded that the extracted functions were not appropriate, a request is made to the characteristic quantity calculating means 19f to modify the specified function to be used. Upon receiving this request, the characteristic quantity calculating means 19f selects a different specified function and calculates characteristic quantities again. As the characteristic functions are changed, the characteristic quantities numerized based on the modified specified function also change and the statistical quantities are also changed. As this process is repeated, the judgment formula determining part 19k selects whichever is provided with satisfactory conditions.

Various conditions may be considered by the judgment formula determining part 19k. For example, the maximum value may be set equal to (normal average value) + $3\sigma$ and the minimum value may be set equal to (average abnormal value) - $3\sigma$.

If the judgment model creating means 19 uses the normal-abnormal comparison method as described above, it defines the selected specified function for the judgment execution means 21.

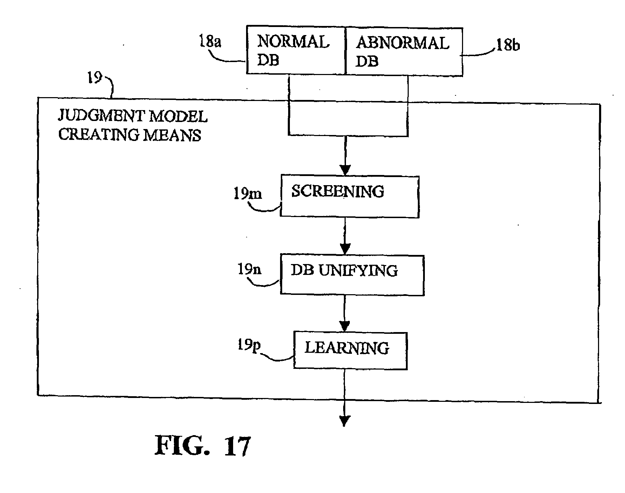

Fig. 17 shows still another structure of the judgment model creating means 19 adapted for the neural network method. By this method, characteristic data for each history type stored in the normal database 18a and the abnormal database 18b of the time-division database 18 are transmitted to a screening means 19m, which serves to calculate an exclusion value for each database and to delete its data. Examples of the exclusion include: (1) those outside (average value) $\pm$ $\sigma$; and (2) those outside (average value) $\pm$ $\sigma$ where the average and standard variation values are calculated by excluding the three largest values (including the maximum) and the three smallest values (including the minimum).

The data remaining after the screening by the screening means 19m are transmitted to a unifying means 19n to unify the data. A learning means 19p then serves to learn a neural network model (to build a clustering model) having the characteristic quantity data stored in the unified database as the input and the history level as the output. Various methods used in the neural network may be used for the learning process. As this process is finished, the neural network model as the result of this learning process is defined as the condition judging means and set in the judgment execution means 21.

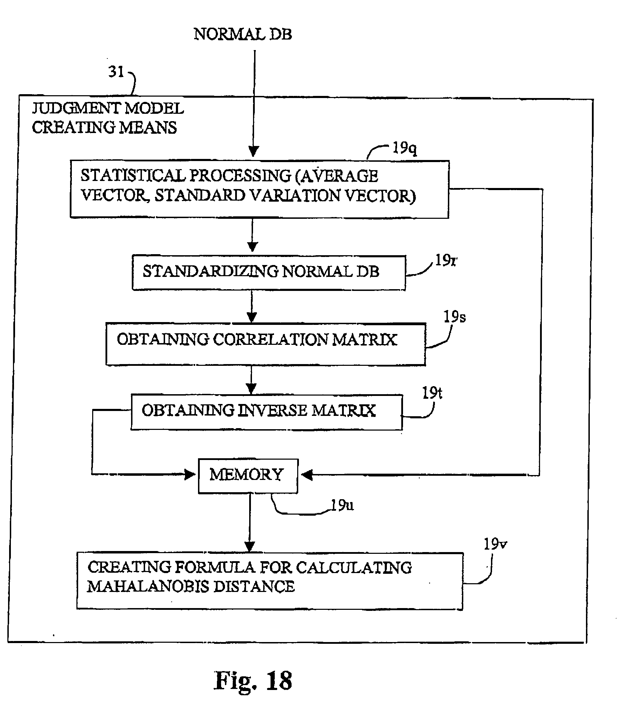

Fig. 18 shows still another structure of the judgment model creating means 19 adapted to unify a plurality of characteristic quantities based only on normal data and to create a judgment model by using the Mahalanobis distance. Characteristic quantities of the normal data stored in the normal database 18a are transmitted to a statistical processing means 19q, which calculates the statistical quantities of all characteristic quantities. Average and standard deviation are the statistical quantities to be obtained. In other words, the average value and the standard variation are obtained for each characteristic quantity and an average vector that lumps together the average values of the characteristic quantities and a standard variation vector that lumps together the standard variations of the characteristic quantities are obtained.

The obtained statistical quantities are transmitted to standardizing means 19r for standardizing the data stored in the normal database 18a by using the average vectors and the standard deviation vectors. This is for the purpose of normalization and standardization because there are variations among the numerical sizes of the characteristic quantities. Next, correlation matrices for the individual characteristic quantities are obtained by a matrix obtaining means 19s and the inverse matrices of these correlation matrices are obtained by inverse matrix obtaining means 19t.

These matrices are stored in a memory means 19u and then transmitted to a formula creating means 19v for obtaining a calculation formula for calculating the Mahalanobis distance.

Mahalanobis distance D^2 is calculated as follows. Consider a situation where n-number of data are measured from a target workpiece with the number of characteristic quantities = k and X1, X2, ⋯⋯, Xk being the values of the items. If the averages and standard variations of the individual characteristic quantities are m1, m2, ⋯⋯, mk and σ1, σ2, ⋯⋯ σk, and the elements of the inverse matrix of the correlation matrix is written as *aij*, Mahalanobis distance is defined as follows:

$$ \mathrm{D^{\wedge}2} = 1/k \sum_{ij} aij\{(Xi-mi)/\sigma i\}\{(Xj-mj)/\sigma j\} $$

where the n-number of data must be preliminarily arranged to be of the same history type. This formula is created by a formula creating means 31f and the created formula is set in the judgment execution means 21.

The normal data that are obtained at the beginning and during an early stage of an endurance test have a pattern closely resembling the pattern of ideal normal data and hence are plotted near the evaluation standard. Mahalanobis distance in this case is nearly equal to 1. By contrast, abnormal data that are obtained after a primary damage has occurred are plotted far away from the evaluation standard according to their distance from the pattern of the normal data. Mahalanobis distance in this case becomes accordingly larger. Thus, a judgment between normal and abnormal can be easily made by examining how close Mahalanobis distance is to 1. The function for evaluating the rate of contribution by a specified function to be used to the reliability of Mahalanobis distance and eliminating those with a low contribution rate may be provided.

What a conventional inspection apparatus does is to cut out a portion of data corresponding to a certain time period from waveform data of an entire sampling time, to consider as a frame a group of data obtained by dividing the cut-out data by a certain data number and to extract a plurality of kinds of characteristic quantities in units of frames. Representative calculated values are then obtained for the individual characteristic quantities obtained from all frames for each kind of the characteristic quantities by various methods such as averaging. Thus, the same number of representative calculated values as the number of different kinds of representative quantities are calculated. The normal-abnormal judgment is made by using either all of these representative calculated values or a specified number of selected ones. No matter what may be the number of the representative values that are used for the judgment, a comparison is made in units of characteristic quantities (scalar quantities) for the judgment.

According to this invention, by contrast, characteristic quantities of a plurality of kinds that are obtained are converted into a single vector quantity for a multi-dimensional waveform evaluation. As a natural consequence, the models to be used for the normal-abnormal judgment are vector quantities created by obtaining a plurality of characteristic quantities. Thus, judgments are made by comparing vector quantities based on such models and waveform data of the target object of inspection. If the distance between two vectors is small, it is judged that the waveform data match the model. If this distance is large, it is judged that the waveform data are different from the model.

For the case of only making normal judgments, only one standard model is necessary with one numerical value obtained by unifying characteristic value of a plurality of kinds (a vector quantity for multi-dimensional waveform evaluation) and a normal judgment can be made by obtaining a distance from it. In other words, after a vector quantity is calculated by unifying individual characteristic quantities (representative calculated values based on a plurality of frames), a judgment can be made by single calculation for obtaining a distance. The distance between two vector quantities may be obtained as Mahalanobis distance, Euclid distance or by any other method.

This unification of characteristic quantities may be applicable to the unification of characteristic quantities of a plurality of waveform data. In the case of waveform data from different sensors or different places of measurement, characteristic quantities are not unified as described above but judgments are made individually based on the waveform obtained by each sensor as the source of waveform data and a summary judgment may be made (by concluding, for example, that there is a primary damage if abnormality is found by any one of the sensors). If there are a plurality of sensing data (such as sound, temperature and vibrations), a judgment algorithm may be created by judging each waveform by using any of the methods described above and finally by unifying the judgment results by a judgment algorithm.

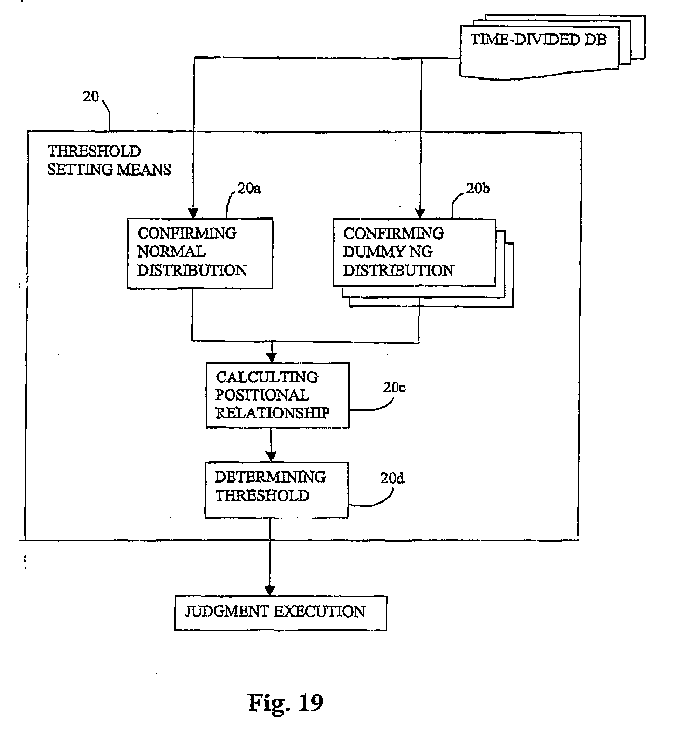

The threshold setting means 20 is for setting a threshold value by a manual operation. As shown in Fig. 19, characteristic quantity data of the normal data stored in the time-division database 18 are transmitted to a normal distribution confirmation means 20a and characteristic quantity data of the abnormal data (dummy NG data) stored in the time-division database 18 are transmitted to a dummy NG distribution confirmation means 20b. If the types of abnormality are set, this is done for each of the abnormality types. In the above, normal and abnormal data may be divided according to the history data or based on a human judgment.

Each of these distribution confirmation means 20a and 20b is for obtaining a distribution condition of characteristic quantities for each of the obtained history type, calculating, for example, average value, center value, standard deviation, quartile point and nσ (where n = 1, 2, ...) and providing the calculated values to a positional relationship calculating means 20c, which serves to obtain positional relationship TX between the normal distribution and one abnormal distribution. For example, positional relationships between the distributions of all abnormality types and the normal distribution such as positional relationship TA between the normal distribution and the distribution of abnormality type A, positional relationship TB between the normal distribution and the distribution of abnormality type B, etc. are obtained.

In the above, positional relationship TX (where X = A, B, C, ...) is a numerical value on characteristic value and may be obtained as follows:

$$TX = (Normal)(Average + 3\sigma) - (Abnormality\ type\ X)(Average\ \text{-}3\sigma).$$

In this formula, (Average) may be replaced by (Center value), "3σ" may be replaced by "Quartile" or by nσ (where n = 1, 2, ⋯).

The positional relationship TX to each abnormal type obtained by this positional relationship calculating means 20c is transmitted to a threshold determining means 20d, which confirms the sign of TX and obtains ΔX according to the rule given as follows.

If TX is negative, this means that the normal distribution and the abnormal distribution are overlapping partially, and ΔX is defined as the middle point of TX as follows:

$$\Delta X = 1/2\{(Normal)\{(Average + 3\sigma) + (Abnormality\ type\ X)(Average\ \text{-}3\sigma)\}.$$

If T=0 or positive, since these distributions are not overlapping, ΔX is set on the side of the distribution of the abnormal type X, or

$$\Delta X = (Abnormality\ type\ X)(Average\ \text{-}3\sigma)\}.$$

After values of ΔX for all abnormal types have been obtained, the smallest of them is defined as the threshold value Δ, or Δ = min (ΔX). As explained above, the average in the above equations may be replaced by the center value or 3σ by the quartile value or by nσ (where n = 1, 2, ⋯).

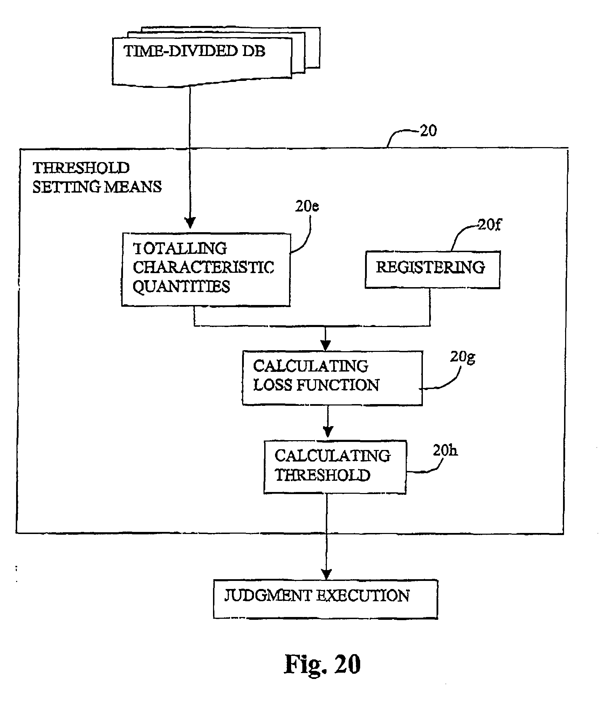

Fig. 20 shows another internal structure of the threshold setting means 20. While both normal and abnormal (dummy NG) distributions are required in the case of Fig. 19, the threshold setting means 20 with the inner structure as shown in Fig. 20 can set a threshold value based only on one of the distributions. Explained more in detail, the data stored in the time-division database 18 are collected and the values of all characteristic quantities of the engine 1 being inspected by the inspection apparatus 10 are totaled, or the standard deviation σ of the characteristic quantity values is obtained.

The threshold setting means 20 shown in Fig. 20 includes a registration means 20f of different kinds for registering, for example, (1) the scrapping cost A0 of the engine 1, (2) the threshold value Δ0 for scrapping decision (level of any characteristic quantity) and (3) the rework cost A of the engine 1. In the above, the scrapping cost means the cost of carrying out the scrapping process as the result of an abnormal judgment, including the cost of production and the cost incurred at the time of scrapping. The rework cost is the cost of replacing components of the engine judged to be abnormal to make a normal product out of it. Characteristic quantities collected by a characteristic quantity collecting means 20e and the registered data inputted to the registration means 20f are transmitted to a loss function calculating means 20g, which serves to calculate a loss function L as follows:

$$L = (A0/\Delta0\hat{\ }s)\sigma\hat{\ }2.$$

The loss function L thus obtained is transmitted to a threshold calculating means 20h on the next stage where threshold value Δ is calculated as follows:

$$\Delta = (A/A0)^{(1/2)} \Delta 0.$$

As explained above, the judgment model is created according to this embodiment only based on data under a normal condition as shown by Steps S4 and S5 of Fig. 2 but data in abnormal condition (dummy NG data) may also be used for creating an even more accurate judgment rule and model.

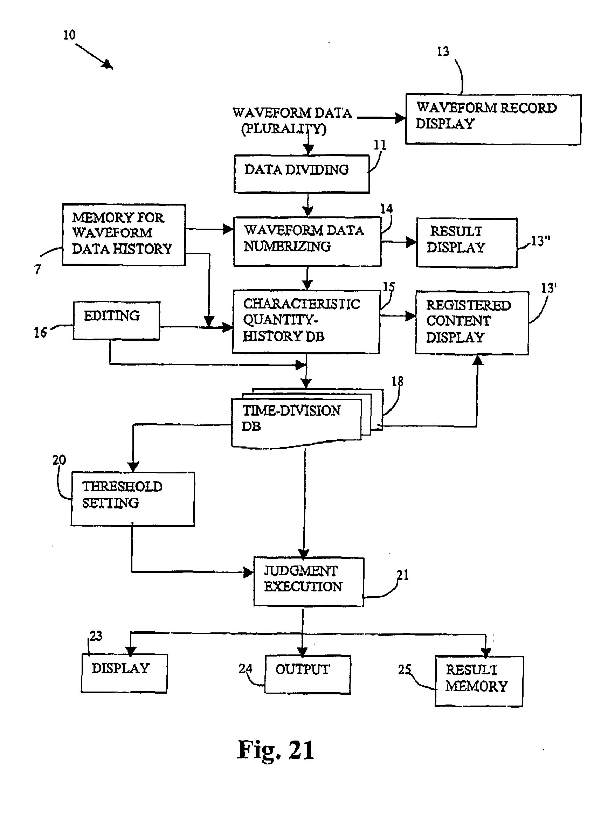

Fig. 21 shows the inner structure of the inspection apparatus 10 at the time of its inspection operation. During this time, the functions for creating a judgment model and adjusting it (or the numerizing means adjusting means 9, the dummy NG creating means 12 and the judgment model creating means 19) are not necessary out of the algorithm shown in Fig. 4. Although the editing means 16 may also be considered unnecessary in view simply of detection of abnormalities, it is kept in order to make manual corrections possible in the case of an error by the inspection apparatus

During an endurance test of the engine 1, as waveform data are continuously inputted from various sensors, the waveform dividing means 11 creates divided waveforms by dividing the waveforms in units ofN seconds and the results are transmitted to the waveform data numerizing means 14. The division process is carried out regularly and sequentially from the beginning. IfN is set randomly at the time of creating the model (algorithm) and divided waveform data are created based thereon, divided waveform data are created for each unit time. Thus, divided waveform data with different unit times (such as 1 second, 2 seconds, 3 seconds, etc.) are created.

The waveform data numerizing means 14 obtains characteristic quantities by numerizing the divided waveform data created from the sensing data obtained during the endurance test of the engine 1 and has these characteristic quantities stored in the characteristic quantity-history database 15. If judgments by human have simultaneously been made on the same target object, the results of such human judgments may also be stored in the characteristic quantity-history database 15. In the case of an endurance test during which the engine 1 is continuously operated for an extended period of time, however, it is not practical to require an inspector to be kept and to continuously input his/her judgments. If such judgments are recorded at an appropriate timing (say, periodically) and registered as a supplement, they may be useful in the subsequent analyses and judgments on the accuracy of the judgment model of the inspection apparatus 10.

The characteristic quantity data stored in the characteristic quantity-history database 15 are transmitted to the judgment execution means 21 where condition judgment (abnormality judgment) is carried out. The results of this judgment are displayed by the display means 23, outputted to the output means 24 and/or stored in the result memory 25.

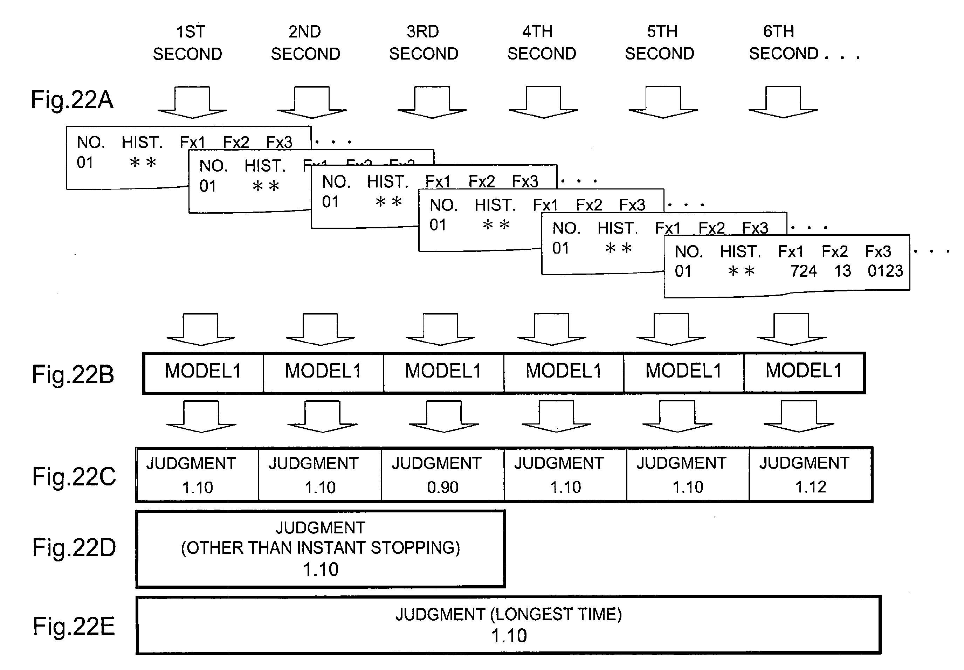

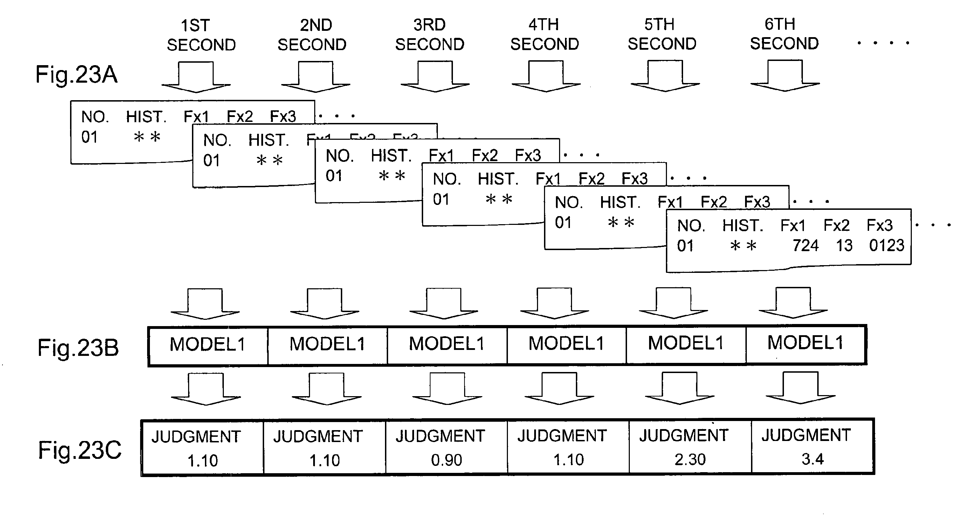

After an algorithm (judgment model) has been created, a test profile is carried out repeatedly as the real endurance test. Abnormalities are detected on real time by evaluating normalcy from the beginning time (0th second) to the end time (Xth second) in units of N seconds. In the example shown in Fig. 22, N = 1 and numerized results (characteristic quantities in the form of a row vector as shown in Fig. 23A) are obtained from waveforms each corresponding to a period of one second. Such row vector data are created continuously in units of one second during the test.

Fig. 22 shows an example of a rotary motion at a constant speed, and hence a single judgment model (Model 1) is used (as shown in Fig. 22B). Normalcy is calculated for each divided waveform data, as shown in Fig. 22C. The judgment model is created such that normalcy approaches 1 as the data approach normal data and becomes larger as the distance from normal data increases. In the case of an endurance test, normalcy is usually nearly equal to 1.0 during an initial period of the test.

The unit time (N) is set not only to 1 second (as required for instant stopping) but also to different times such as 3 seconds and 6 seconds. Normalcy is calculated also based on such divided waveform data, as shown in Figs. 22D and 22E.

Fig. 23 shows another example where the normalcy judgment result is nearly equal to 1 until the fifth second but it is as large as 2.3 in the fifth second and it is still larger and 3.4 in the sixth second, quickly moving away from 1. If the threshold value for abnormal judgment is less than 2.3, a stop command is outputted at the time of judgment in the fifth second. The endurance test for the engine 1 is thereby stopped and waveform data for the sixth second are not inputted and hence there is no judgment result. (The threshold value is not less than 2.3 in Fig. 23 and this is why judgment results are outputted until the sixth second.)

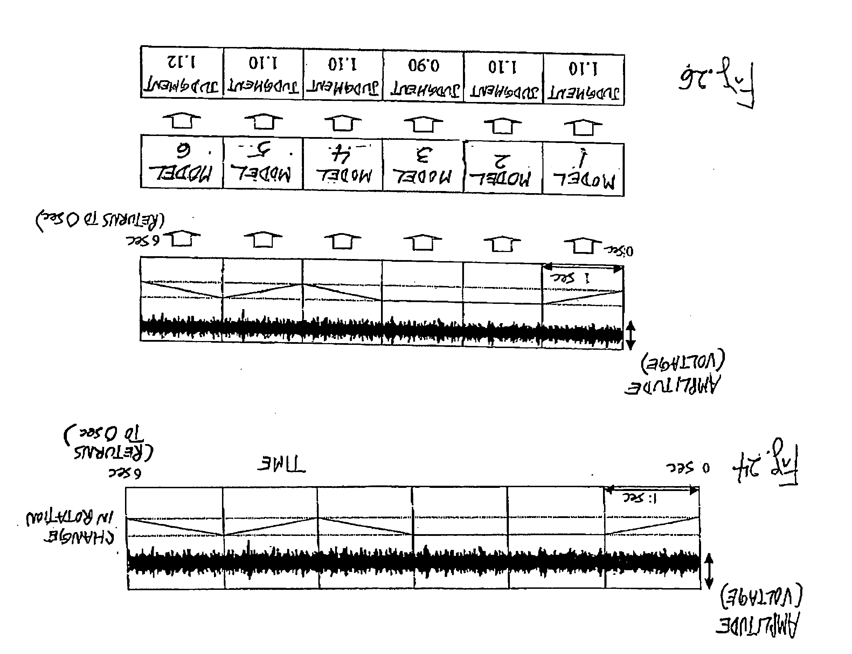

Examples of a constant-speed motion were described above and hence a common judgment model was used for each of divided waveform data but the invention is naturally not limited to such applications but is also applicable to operation profiles with a variable speed. Fig. 24 shows another example wherein the rotation of the engine 1 is accelerated during the first second, at a constant rate during the second and third seconds and changed alternately between deceleration and acceleration thereafter.

The judgment model (algorithm) in such a situation is created firstly by dividing the normal waveform data obtained during an initial period in units of one second by the waveform dividing means 11. If the rate of rotation changes quickly as in this example, the random change of initial time of division is not carried out although the method of randomly changing the beginning time of division may be used if the same type of motion continues for a certain fixed period of time. Although Fig. 24 presents an example wherein the kind of motion remains the same within each period of one second, this is not always the situation. If an acceleration lasts for only 0.5 seconds, followed by a constant-speed motion for another period of 0.5 seconds, for example, such a change can be extracted as a characteristic of this waveform portion, there is no problem if a judgment is made by matching the timing appropriately.

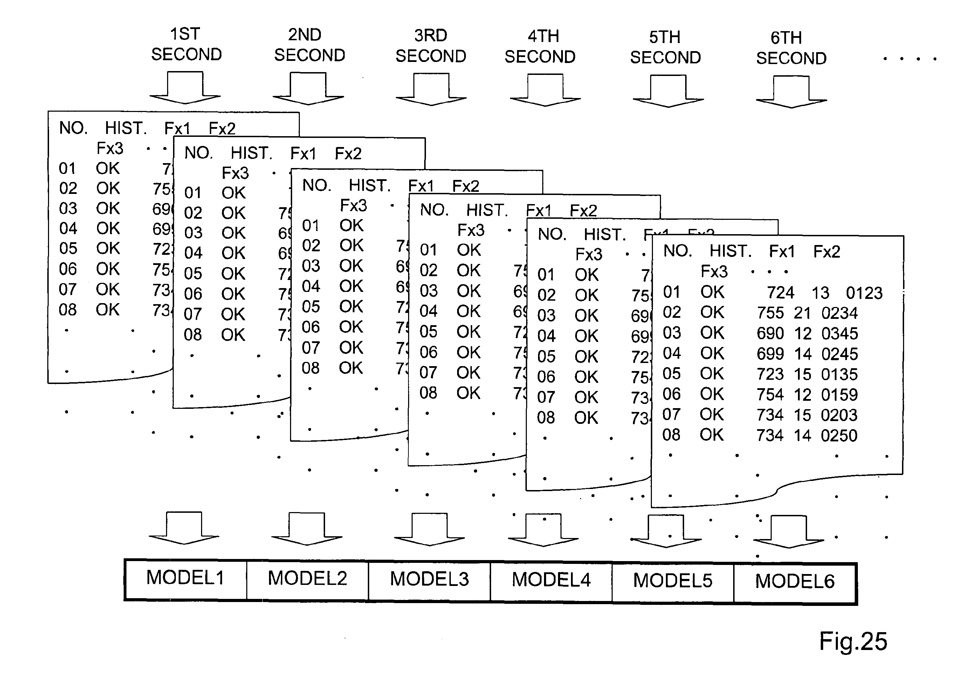

Next, the waveform data numerizing means 14 obtains characteristic quantities by a frame-division process on the obtained divided waveform data. The frame-division process may be carried out by varying the frame sizes at random such that an accurate judgment model can be created although only waveform data corresponding to one second can be obtained. In an actual operation, a test profile such as shown in Fig. 24 may be repeated several times for creating a judgment model to collect waveform data of the same timing such that a more accurate judgment algorithm (judgment model) can be created. Fig. 25 shows an example wherein waveform data are collected 8 times for each divided waveform data, and this is further repeated to obtain a sufficient number of data for creating a model. Judgment models 1-6 are thus created in this example from the first second to the sixth second.

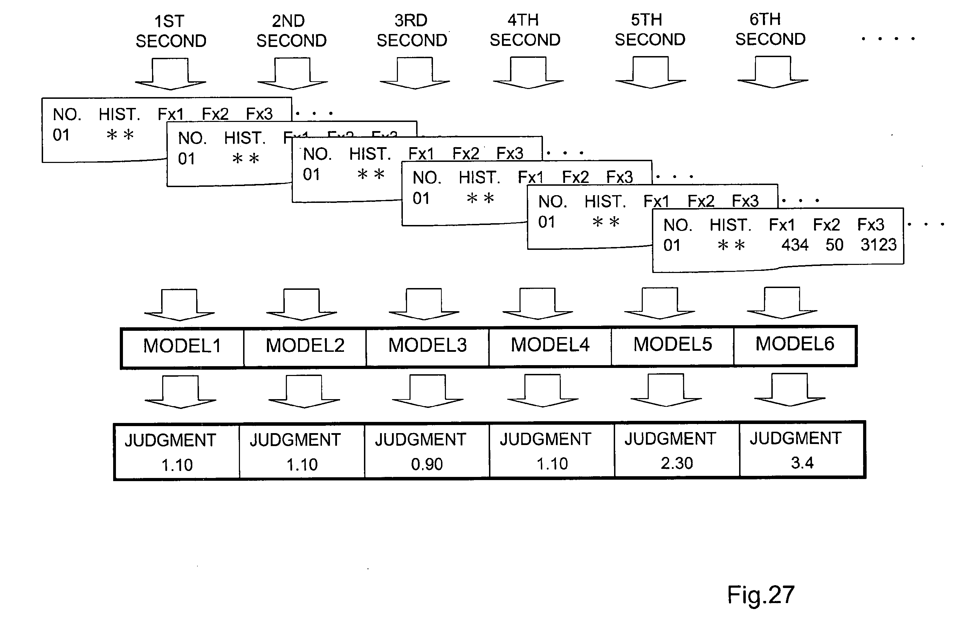

After Models 1- 6 are created corresponding to the first through sixth seconds, an actual judgment process is carried out for the endurance test by using the judgment model corresponding to each condition of the operation profile. Each divided waveform data can be obtained accurately by receiving timing signals from the PLC 3. If the judgment result is as shown in Fig. 26, it may be concluded that the situation is normal since the judgment value is nearly equal to 1 by each of the judgment models.

If the test continues for a long time, the result may be as shown in Fig. 27, the judgment result based on Judgment Model 5 for the fifth second becoming as large as 2.3 and that based on Judgment Model 6 for the sixth second becoming even larger and 3.4, as in the case of Fig. 23. In this case, it is not clear whether the distance is increasing gradually from 1 or a primary damage is occurring in different components between the time of acceleration (during the fifth second) and that of deceleration (during the sixth second) but there is no doubt in either case that some weakened component or components are damaged or about to be damaged.

If the threshold value for the judgment of abnormality is less than 2.3 for every judgment model, a stop command is outputted at the time of judgment by Model 5 and the endurance test of the engine 1 is stopped and the waveform data for the sixth second are not inputted or processed. Fig. 27 indicates that the threshold value was not less than 2. 3 and this is why a judgment is made for the sixth second. Different threshold values may be set for different judgment models.

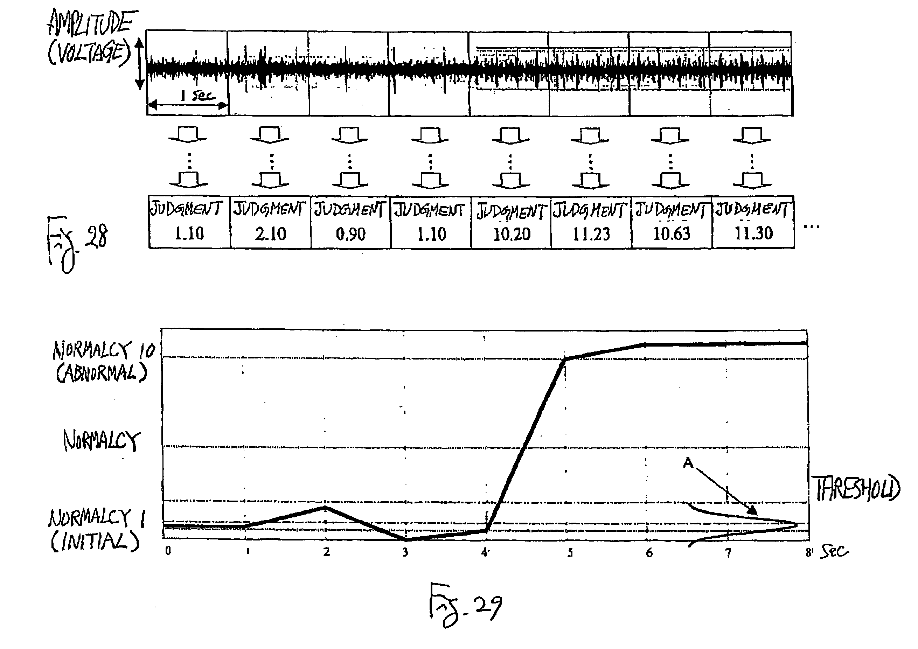

Figs. 28 and 29 show examples of display of change in normalcy or a method of using a endurance test apparatus. This example shows that a slight abnormality was detected 2 seconds after the beginning of the test such that the judgment value rose to 2.1 (such as a momentary change in the tone of the sound) and a normal condition returned immediately afterwards but the judgment value changed suddenly to a larger value over 10. In such a case, it is up to the user to decide whether or not the light abnormality with the judgment value of 2.1 should be treated as a formal abnormality for instantly stopping. The slight abnormality experienced first could be a precursor of a primary damage or a mere fluctuation during a normal operation. Thus, a threshold value can be calculated by studying the lower slopes of the distribution curve of normalcy at the normal time (initial condition). For example, if the normal range may be defined as being within $\pm 3\sigma$ of the average, 99.7% will be included within this range and hence the range beyond may be defined as being abnormal.

Although Fig. 1 showed an example of endurance test with sensors adapted to output waveform data as analog signals that are converted by the AD converter 6 to be transmitted to the inspection apparatus 10, these sensors themselves may contain a data processor and be adapted to output digital signals or to output waveform data to the inspection apparatus 10 though a DD converter. Depending on the capability of various data devices, some of the functions of the inspection apparatus 10 may be carried out by such a data processor.

In the example shown in Fig. 1, there was only one target object for the endurance test but there are many situations where endurance tests are concurrently carried out on a plurality of target objects. In such a situation, since it is not only inefficient but also undesirable from the point of view of cost to connect an inspection apparatus to each of the target objects, it may be so arranged that only one inspection apparatus 10 is used to monitor a plurality of endurance tests. If the engines 1 and their driving devices 2 are of the same types in such a situation, a single judgment model may be used in common but it is preferable to create a judgment model and algorithm for each test based on the normal data obtained during its initial period immediately after its beginning and to make individual judgments. This is because although the targets may be of the same type, it is necessary to consider the effects of environmental conditions and there are expected to be some fluctuations among the target objects.

Although examples were shown above wherein the inspection apparatus were set where the endurance test was taking place, this is not intended to limit the scope of the invention. An inspection apparatus may be located at some distant place far away and connected with a computer at the place of testing (serving as data collecting means) with a dedicated high-speed line or a network system of some other kind such that real-time waveform data can be obtained by the inspection apparatus to carry out judgments and to output a stop signal if necessary by using the same or different communication network. Especially where a work is necessary for setting and adjustment in creating a judgment model, the worker is not required to travel to the place of testing and can complete the necessary work at a monitoring center. Data collecting means such as a data logger may be used for collection of data.

In summary, the specification is intended to be interpreted broadly. Many modifications and variations possible within the scope of the invention, and such modifications and variations that are obvious to a person skilled in the art are intended to be within the scope of the invention.

## Claims

1. An aid device for creating judgment model for an inspection apparatus for extracting characteristic quantities from waveform data obtained from a target object of inspection and judging condition of said target object based on the extracted characteristic quantities, said aid device comprising:

   divided waveform data creating means for creating divided waveform data by dividing obtained waveform data into portions of unit time;
   calculating means for obtaining characteristic quantities in units of the divided waveform data based on a plurality of the divided waveform data for the unit time; and
   model creating means for creating judgment model for judging condition of said target object for the unit time based on the obtained characteristic quantities.

2. The aid device of claim 1 wherein said divided waveform data creating means serves to divide waveform data into portions of said unit time from a specified position.

3. The aid device of claim 1 wherein said divided waveform data creating means serves to determine said specified position at random.

4. The aid device of claim 2 wherein said divided waveform data creating means serves to determine said specified position at random.

5. The aid device of claim 1 wherein said divided waveform data creating means serves to determine said unit time at random.

6. The aid device of claim 2 wherein said divided waveform data creating means serves to determine said unit time at random.

7. The aid device of claim 3 wherein said divided waveform data creating means serves to determine said unit time at random.

8. The aid device of claim 4 wherein said divided waveform data creating means serves to determine said unit time at random.

9. An aid device for creating judgment model for an inspection apparatus for extracting characteristic quantities from waveform data obtained from a target object of inspection operating repeatedly in a specified pattern and judging condition of said target object based on the extracted characteristic quantities, said aid device comprising:

   divided waveform data creating means for creating divided waveform data by dividing obtained waveform data into portions of unit time;
   calculating means for obtaining characteristic quantities in units of the divided waveform data based on a plurality of the divided waveform data for the unit time; and
   model creating means for creating judgment model, based on the obtained characteristic quantities, for each of areas into which said pattern has been divided by said divided waveform data creating means.

10. The aid device of claim 1 wherein said calculating means has the functions of:

    carrying out a frame division process by further dividing said divided waveform data into frames of a specified size;
    extracting said characteristics quantities based on said divided frames; and determining said specified size at random.

**11.** The aid device of claim 9 wherein said calculating means has the functions of:

carrying out a frame division process by further dividing said divided waveform data into frames of a specified size;
extracting said characteristics quantities based on said divided frames; and
determining said specified size at random.

**12.** The aid device of claim 10 wherein said calculating means further has the functions of carrying out a frequency analysis on each of said frames obtained by carrying out said frame division process.

**13.** The aid device of claim 11 wherein said calculating means further has the functions of carrying out a frequency analysis on each of said frames obtained by carrying out said frame division process.

**14.** An inspection apparatus comprising judging means for carrying out a status judgment on obtained target object of inspection based on a judgment model created by an aid device, said aid device comprising:

divided waveform data creating means for creating divided waveform data by dividing obtained waveform data into portions of unit time;
calculating means for obtaining characteristic quantities in units of the divided waveform data based on a plurality of the divided waveform data for the unit time;
model creating means for creating judgment model for judging condition of said target object for the unit time based on the obtained characteristic quantities.

**15.** An inspection apparatus for inspecting a target object that operates by repeating specified patterns, said inspection apparatus comprising judging means having the functions of:

obtaining judgment models based on one of said repeated specified pattern by using an aid device, said aid device comprising:

divided waveform data creating means for creating divided waveform data by dividing obtained waveform data into portions of unit time;
calculating means for obtaining characteristic quantities in units of the divided waveform data based on a plurality of the divided waveform data for the unit time; and
model creating means for creating judgment model, based on the obtained characteristic quantities, for each of areas into which said pattern has been divided by said divided waveform data creating means; and

carrying out a judgment process by using said obtained judgment models corresponding to obtained waveform portions.

**16.** An endurance test apparatus for carrying out an endurance test to test endurance of a target object; said endurance test apparatus comprising judgment means for judging whether said target object is normal or abnormal by using a judgment model that is created by using, as normal data, data obtained during an initial period of said endurance test when said endurance test apparatus is operating stably and based on waveform data obtained during said endurance test.

**17.** The endurance test apparatus of claim 16 wherein said judgment mode is created by using an aid device that comprises:

divided waveform data creating means for creating divided waveform data by dividing obtained waveform data into portions of unit time;
calculating means for obtaining characteristic quantities in units of the divided waveform data based on a plurality of the divided waveform data for the unit time;
model creating means for creating judgment model for judging condition of said target object for the unit time based on the obtained characteristic quantities.

**18.** The endurance test apparatus of claim 16 wherein said judgment mode is created by using aid device comprising:

divided waveform data creating means for creating divided waveform data by dividing obtained waveform data into portions of unit time;

calculating means for obtaining characteristic quantities in units of the divided waveform data based on a plurality of the divided waveform data for the unit time; and

model creating means for creating judgment model, based on the obtained characteristic quantities, for each of areas into which a pattern of operation of said endurance test apparatus has been divided by said divided waveform data creating means.

**19.** An endurance test method comprising the steps of:

causing a rotary motion for a specified length of time and providing waveform data obtained meanwhile to an aid device that comprises:

divided waveform data creating means for creating divided waveform data by dividing obtained waveform data into portions of unit time;

calculating means for obtaining characteristic quantities in units of the divided waveform data based on a plurality of the divided waveform data for the unit time; and

model creating means for creating judgment model for judging condition of said target object for the unit time based on the obtained characteristic quantities;

causing said aid device to create judgment models by using said provided waveform data as normal data; thereafter carrying out an endurance test to judge presence and absence of abnormality based on waveform data that are obtained during said endurance test by using said created judgment models; and

outputting an alarm signal if abnormality is detected.

**20.** An endurance test method comprising the steps of:

causing a rotary motion for a specified length of time and providing waveform data obtained meanwhile to an aid device that comprises:

divided waveform data creating means for creating divided waveform data by dividing obtained waveform data into portions of unit time;

calculating means for obtaining characteristic quantities in units of the divided waveform data based on a plurality of the divided waveform data for the unit time; and

model creating means for creating judgment model, based on the obtained characteristic quantities, for each of areas into which a pattern of operation of said endurance test apparatus has been divided by said divided waveform data creating means;

causing said aid device to create judgment models by using said provided waveform data as normal data; thereafter carrying out an endurance test to judge presence and absence of abnormality based on waveform data that are obtained during said endurance test by using said created judgment models; and

outputting an alarm signal if abnormality is detected.

Fig.1

CREATION OF MONITORING ALGORITHM

S1 — ATTACH SENSOR TO WORKPIECE

S2 — START ENDURANCE TEST

S3 — STOP ENDURANCE TEST

S4 — TRANSMIT OBTAINED SENSING DATA TO JUDGMENT ALGORITHM CREATING MEANS AS NORMAL DATA

S5 — NUMERIZE SENSING DATA TO CREATE STANDARD SPACE AND REGISTER

EXECUTION OF ENDURANCE TEST

S6 — SET THRESHOLD VALUE

S7 — RESTART ENDURANCE TEST

S8 — SENSING DATA TO INSPECTION APPARATUS FOR REAL-TIME CONDITION JUDGMENT

S9 — STOP COMMAND TO TEST APPARATUS IF THRESHOLD VALUE IS EXCEEDED

Fig. 2

AMPLITUDE
(VOLTAGE)

1 SEC

Fig. 3A
(PRIOR ART)

MODEL

AMPLITUDE
(VOLTAGE)

1 SEC

Fig. 3B

| MODEL 1 | MODEL 2 | MODEL 3 | MODEL 4 | MODEL 5 | MODEL 6 |

EP 1 705 468 A2

Fig. 4

AMPLITUDE
(VOLTAGE)

1 Sec.     1 Sec     1 Sec

0 Sec                                    6 Sec

Fig. 5

AMPLITUDE
(VOLTAGE)

2 Sec

0 Sec                                    6 Sec

Fig. 6

24

WAVEFORM DATA
(PLURALITY)

12

DUMMY NG
CREATING MEANS

WAVEFORM DEFORMATION
PARAMETER SETTING                12a

DEFORMATION
METHOD SETTING                   12b

WAVEFORM DATA
SELECTING                        12c

                                 12d
WAVEFORM DEFORMING

DUMMY NG WAVEFORM CREATING (PLURALITY)

# Fig. 7

| NO | HIST | Fx1 | Fx2 | Fx3 | ··· |
|----|------|-----|-----|-----|-----|
| 01 | OK | 724 | 13 | 0123 | ··· |
| 02 | OK | 755 | 21 | 0234 | ··· |
| 03 | OK | 690 | 12 | 0345 | ··· |
| 04 | OK | 699 | 14 | 0245 | ··· |
| 05 | OK | 723 | 15 | 0135 | ··· |
| 06 | OK | 754 | 12 | 0159 | ··· |

TIME

Fig. 8

AMPLITUDE
(VOLTAGE)

i sec

off

T1   T2   T3                                    Tm

1 sec

Fig - 9

T1  T2  T3                                     Tm

FFT                                            1 sec

TIME (FRAME)                    (TIME DIRECTION)

T1      T2      T3          Tm        AVERAGE  STANDARD DEVIATION

| | T1 | T2 | T3 | | Tm | | | |
|---|---|---|---|---|---|---|---|---|
| F1 | $P_{F1}(T1)$ | $P_{F1}(T2)$ | $P_{F1}(T3)$ | | $P_{F1}(Tm)$ | | $\mu_{F1}$ | $\sigma_{F1}$ |
| F2 | $P_{F2}(T1)$ | $P_{F2}(T2)$ | $P_{F2}(T3)$ | | $P_{F2}(Tm)$ | | $\mu_{F2}$ | $\sigma_{F2}$ |
| F3 | $P_{F3}(T1)$ | $P_{F3}(T2)$ | $P_{F3}(T3)$ | | $P_{F3}(Tm)$ | | $\mu_{F3}$ | $\sigma_{F3}$ |
| | | | | | | | | |
| | | | | | | | | |
| Fn | $P_{Fn}(T1)$ | $P_{Fn}(T2)$ | $P_{Fn}(T3)$ | | $P_{Fn}(Tm)$ | | $\mu_{Fn}$ | $\sigma_{Fn}$ |

FREQUENCY

Fig. 10

CHARACTERISTIC QUANTITY(1)

AVERAGES

| $\mu$ F1 | $\mu$ F2 | $\mu$ F3 | | | $\mu$ Fn |
|---|---|---|---|---|---|

STANDARD DEVIATIONS

| $\sigma$ F1 | $\sigma$ F2 | $\sigma$ F3 | | | $\sigma$ Fn |
|---|---|---|---|---|---|

· · ·

CHARACTERISTIC QUANTITY(n)

AVERAGES

· ·

| $\mu$ F1 | $\mu$ F2 | $\mu$ F3 | | | $\mu$ Fn |
|---|---|---|---|---|---|

STANDARD DEVIATIONS

| $\sigma$ F1 | $\sigma$ F2 | $\sigma$ F3 | | | $\sigma$ Fn |
|---|---|---|---|---|---|

Fig.11

EP 1 705 468 A2

Fig. 12

WAVEFORM DATA (PLURALITY)

14

FIRST TEMPLATE MEMORY 　14a

FRAME DIVISION 　14f

FREQUENCY ANALYSIS 　14b

WAVEFORM DATA HISTORY RECORDING MEANS

14c SECOND TEMPLATE MEMORY

NUMRIZING MEANS ADJUSTING MEANS

14d FUNCTION OPTIMIZING

14e NUMERIZATION

WAVEFORM DATA NUMERIZING MEANS

Fig. 13

START

S11

SET CONDITIONS FOR CHECKING EFFECTS OF
COMBINATIONS OF COEFFICIENTS AND
CONSTANTS OF SPECIFIED FUNCTION BASED ON
PLANNING LIST

S12

CALCULATE DYNAMIC CHARACTERISTIC (SN RATIO) FOR
EACH INVESTIGATION METHOD (EXPERIMENT NO.) WITH
NORMAL AND ABNORMAL AS SIGNAL ELEMENTS AND DATA
NUMBER AS ERROR ELEMENT

S13

CALCULATE PROCESS AVERAGES OF COEFFICIENTS AND
CONSTANTS OF SPECIFIED FUNCTION

S14

SELECT VALUE WITH HIGH SN RATIO FOR EACH
COEFFICIENT AND CONSTANT OF SPECIFIED FUNCTION

S15

SET COEFFICIENTS AND CONSTANTS OF
SPECIFIED FUNCTION TO OPTIMUM VALUES

**FIG. 14**

END

NORMAL
DB          18a

ABNORMAL
DB          18b

19

JUDGMENT MODEL
CREATING MEANS

CALCULATING AND
SAVING EUCLID
DISTANCES OF
NORMAL DATA          19a

CALCULATING AND
SAVING EUCLID
DISTANCES OF
ABNORMAL DATA          19b

CALCULATING NORMAL
STATISTICAL
QUANTITIES          19c

CALCULATING
ABNORMAL STATISTICAL
QUANTITIES          19d

DETERMINING JUDGMENT FORMULA          19e

## Fig. 15

EP 1 705 468 A2

```
        ┌──────────┬──────────┐
   18a  │ NORMAL   │ ABNORMAL │  18b
        │ DB       │ DB       │
        └──────────┴──────────┘

┌─────────────────────────────────────────────────────────────┐
│ JUDGMENT MODEL                                                │
│ CREATING MEANS                                    ⌐19f        │
│                                                               │
│  ┌─────────────────────────────────────────────────────┐     │
│  │ CALCULATING CHARACTERISTIC QUANTITY                  │◄──┐ │
│  └─────────────────────────────────────────────────────┘   │ │
│                                                            │ │
│  19g                                               19h     │ │
│  ┌──────────────────┐          ┌──────────────────┐        │ │
│  │ STORING NORMAL   │◄────────►│ STORING ABNORMAL │        │ │
│  │ CHARACTERISTIC   │          │ CHARACTERISTIC   │        │ │
│  │ QUANTITY DATA    │          │ QUANTITY DATA    │        │ │
│  └──────────────────┘          └──────────────────┘        │ │
│                                                     ⌐19j    │ │
│  19i                                                        │ │
│  ┌──────────────────┐          ┌──────────────────┐        │ │
│  │ CALCULATING      │          │ CALCULATING      │        │ │
│  │ NORMAL STATISTICAL│         │ ABNORMAL STATISTICAL│      │ │
│  │ QUANTITIES       │          │ QUANTITIES       │        │ │
│  └──────────────────┘          └──────────────────┘        │ │
│                                                            │ │
│  19k     ┌────────────────────────────────────────┐        │ │
│          │ DETERMINING JUDGMENT FORMULA            │────────┘ │
│          └────────────────────────────────────────┘          │
│                                                               │
└───────────────────────────────────────────────────────────────┘
```

## FIG. 16

FIG. 17

NORMAL DB

31 —

JUDGMENT MODEL
CREATING MEANS

╭─ 19q
STATISTICAL PROCESSING (AVERAGE
VECTOR, STANDARD VARIATION VECTOR)

STANDARDIZING NORMAL DB   ╱ 19r

OBTAINING CORRELATION MATRIX   ╱ 19s

OBTAINING INVERSE MATRIX   19t

MEMORY

╲ 19u

CREATING FORMULA FOR CALCULATING   19v
MAHALANOBIS DISTANCE

## Fig. 18

TIME-DIVIDED DB

20

THRESHOLD
SETTING MEANS

20a

CONFIRMING
NORMAL
DISTRIBUTION

20b

CONFIRMING
DUMMY NG
DISTRIBUTION

CALCULTING
POSITIONAL
RELATIONSHIP

20c

DETERMINING
THRESHOLD

20d

JUDGMENT EXECUTION

**Fig. 19**

TIME-DIVIDED DB

—20

THRESHOLD
SETTING MEANS

20e

TOTALLING
CHARACTERISTIC
QUANTITIES

20f

REGISTERING

CALCULATING
LOSS FUNCTION

20g

CALCULATING
THRESHOLD

20h

JUDGMENT EXECUTION

**Fig. 20**

**Fig. 21**

EP 1 705 468 A2

|  | 1ST SECOND | 2ND SECOND | 3RD SECOND | 4TH SECOND | 5TH SECOND | 6TH SECOND . . . |
|---|---|---|---|---|---|---|

Fig.22A

| NO. | HIST. | Fx1 | Fx2 | Fx3 | . . . |
|---|---|---|---|---|---|
| 01 | ＊＊ | | | | |

| NO. | HIST. | Fx1 | Fx2 | Fx3 | . . . |
|---|---|---|---|---|---|
| 01 | ＊＊ | | | | |

| NO. | HIST. | Fx1 | Fx2 | Fx3 | . . . |
|---|---|---|---|---|---|
| 01 | ＊＊ | | | | |

| NO. | HIST. | Fx1 | Fx2 | Fx3 | . . . |
|---|---|---|---|---|---|
| 01 | ＊＊ | | | | |

| NO. | HIST. | Fx1 | Fx2 | Fx3 | . . . |
|---|---|---|---|---|---|
| 01 | ＊＊ | 724 | 13 | 0123 | |

Fig.22B

| MODEL1 | MODEL1 | MODEL1 | MODEL1 | MODEL1 | MODEL1 |
|---|---|---|---|---|---|

Fig.22C

| JUDGMENT 1.10 | JUDGMENT 1.10 | JUDGMENT 0.90 | JUDGMENT 1.10 | JUDGMENT 1.10 | JUDGMENT 1.12 |
|---|---|---|---|---|---|

Fig.22D

| JUDGMENT (OTHER THAN INSTANT STOPPING) 1.10 |
|---|

Fig.22E

| JUDGMENT (LONGEST TIME) 1.10 |
|---|

EP 1 705 468 A2

Fig.23A

| 1ST SECOND | 2ND SECOND | 3RD SECOND | 4TH SECOND | 5TH SECOND | 6TH SECOND | · · · · |
|---|---|---|---|---|---|---|

| NO. | HIST. | Fx1 | Fx2 | Fx3 | · · · |
|---|---|---|---|---|---|
| 01 | ∗∗ | | | | |

| NO. | HIST. | Fx1 | Fx2 | Fx3 | · · · |
|---|---|---|---|---|---|
| 01 | ∗∗ | | | | |

| NO. | HIST. | Fx1 | Fx2 | Fx3 | · · · |
|---|---|---|---|---|---|
| 01 | ∗∗ | | | | |

| NO. | HIST. | Fx1 | Fx2 | Fx3 | · · · |
|---|---|---|---|---|---|
| 01 | ∗∗ | | | | |

| NO. | HIST. | Fx1 | Fx2 | Fx3 | · · · |
|---|---|---|---|---|---|
| 01 | ∗∗ | | | | |

| NO. | HIST. | Fx1 | Fx2 | Fx3 | · · · |
|---|---|---|---|---|---|
| 01 | ∗∗ | 724 | 13 | 0123 | |

Fig.23B

| MODEL1 | MODEL1 | MODEL1 | MODEL1 | MODEL1 | MODEL1 |
|---|---|---|---|---|---|

Fig.23C

| JUDGMENT 1.10 | JUDGMENT 1.10 | JUDGMENT 0.90 | JUDGMENT 1.10 | JUDGMENT 2.30 | JUDGMENT 3.4 |
|---|---|---|---|---|---|

EP 1 705 468 A2

AMPLITUDE
(VOLTAGE)

CHANGE
IN ROTATION

1 sec

Fig. 24

0 sec

TIME

6 sec
(RETURNS
TO 0 sec)

AMPLITUDE
(VOLTAGE)

1 sec

0 sec

6 sec
(RETURNS TO 0 sec)

| MODEL 1 | MODEL 2 | MODEL 3 | MODEL 4 | MODEL 5 | MODEL 6 |
|---------|---------|---------|---------|---------|---------|
| JUDGMENT 1.10 | JUDGMENT 1.10 | JUDGMENT 0.90 | JUDGMENT 1.10 | JUDGMENT 1.10 | JUDGMENT 1.12 |

Fig. 26

1ST SECOND  2ND SECOND  3RD SECOND  4TH SECOND  5TH SECOND  6TH SECOND · · · ·

| NO. | HIST. | Fx1 | Fx2 | Fx3 | · · · |
|---|---|---|---|---|---|
| 01 | OK | 724 | 13 | 0123 | |
| 02 | OK | 755 | 21 | 0234 | |
| 03 | OK | 690 | 12 | 0345 | |
| 04 | OK | 699 | 14 | 0245 | |
| 05 | OK | 723 | 15 | 0135 | |
| 06 | OK | 754 | 12 | 0159 | |
| 07 | OK | 734 | 15 | 0203 | |
| 08 | OK | 734 | 14 | 0250 | |

| MODEL1 | MODEL2 | MODEL3 | MODEL4 | MODEL5 | MODEL6 |
|---|---|---|---|---|---|

Fig.25

1ST SECOND   2ND SECOND   3RD SECOND   4TH SECOND   5TH SECOND   6TH SECOND   · · · ·

| NO. | HIST. | Fx1 | Fx2 | Fx3 | · · · |
|-----|-------|-----|-----|-----|-------|
| 01  | * *   |     |     |     |       |

| NO. | HIST. | Fx1 | Fx2 | Fx3 | · · · |
|-----|-------|-----|-----|-----|-------|
| 01  | * *   |     |     |     |       |

| NO. | HIST. | Fx1 | Fx2 | Fx3 | · · · |
|-----|-------|-----|-----|-----|-------|
| 01  | * *   |     |     |     |       |

| NO. | HIST. | Fx1 | Fx2 | Fx3 | · · · |
|-----|-------|-----|-----|-----|-------|
| 01  | * *   |     |     |     |       |

| NO. | HIST. | Fx1 | Fx2 | Fx3 | · · · |
|-----|-------|-----|-----|-----|-------|
| 01  | * *   | 434 | 50  | 3123 |      |

| MODEL1 | MODEL2 | MODEL3 | MODEL4 | MODEL5 | MODEL6 |
|--------|--------|--------|--------|--------|--------|

| JUDGMENT 1.10 | JUDGMENT 1.10 | JUDGMENT 0.90 | JUDGMENT 1.10 | JUDGMENT 2.30 | JUDGMENT 3.4 |
|---------------|---------------|---------------|---------------|---------------|--------------|

Fig.27

AMPLITUDE
(VOLTAGE)

|← 1 sec →|

| JUDGMENT 1.10 | JUDGMENT 2.10 | JUDGMENT 0.90 | JUDGMENT 1.10 | JUDGMENT 10.20 | JUDGMENT 11.23 | JUDGMENT 10.63 | JUDGMENT 11.30 | ... |

Fig. 28

NORMALCY 10
(ABNORMAL)

NORMALCY

NORMALCY 1
(INITIAL)

A

THRESHOLD

0    1    2    3    4    5    6    7    8  Sec

Fig. 29

EP 1 705 468 A2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3484665 B

- JP 3103193 B